# EUROPEAN PATENT APPLICATION

(11) **EP 2 526 885 A1**
(43) Date of publication of application: **28.11.2012**
(21) Application number: 10843883.9
(22) Date of filing: 22.01.2010
(51) Int. Cl.: A61B 18/00

(54) **TREATMENT TOOL, TREATMENT DEVICE, AND TREATMENT METHOD**

(71) Applicant: OLYMPUS MEDICAL SYSTEMS CORP., Tokyo 151-0072 (JP)
(72) Inventor: TAKASHINO, Tomoyuki, Tokyo 192-8512 (JP)
(74) Representative: Schicker, Silvia
(86) International application number: PCT/JP2010/050841
(87) International publication number: WO 2011/089718

(57) **Abstract**

A medical treatment device (12) configured to treat body tissues (L1, L2) for conjugation includes a pair of holding members (52, 54) to hold the body tissues, an energy output portion (92, 94) provided in at least one of the holding members to join the body tissues by supplying energy to the body tissues, and a conjugation coating portion (262) to coat contact surfaces (C1, C2) of the body tissues with a substance capable of preventing fluid from invading.

## Description

### Technical Field

The present invention relates to a medical treatment device, a medical treatment system, and a medical treatment method to cure/treat body tissues.

### Background Art

It is generally known that body tissues can be joined by (1) bringing body tissues to be joined into contact, (2) denaturing proteins of target tissues, and (3) removing fluid present between target tissues. This is bond using a so-called hydrogen bond, which is a linkage using polarity of a polar group of amino acids constituting proteins. Such a description can be found in, for example, U.S. Pat. No. 6626901.

Note that denaturing proteins denotes inducing a conformational change, which is one of features of proteins, that is, dissociating the linkage of polar groups linked with certain regularity to form the conformational structure of proteins. It becomes possible to promote a new linkage with a polar group present in adjacent proteins by using the polar group freed by dissociating the linkage of polar groups and so a linkage of proteins and accordingly, conjugation of body tissues can be induced.

To induce the phenomenon, various forms of energy such as high frequencies, heat, ultrasonic, and laser light are used by medical treatment devices. By using such forms of energy, the temperature of joining target tissues is raised to denature proteins and to remove fluid (H₂O) present between target tissues simultaneously. Conjugation of tissues is thereby achieved. Energy devices currently used as blood vessel sealing devices use this phenomenon.

An effect brought about by removing fluid (H₂O) will be described. It is generally known that a water molecule H₂O has a strong polarity. Due to the strong polarity, the water molecule is known to be easily linked to a polar group having a polarity. The linkage is also established between water molecules H₂O, thereby inducing a phenomenon specific to water molecules H₂O. For example, while the heat of vaporization of helium is 0.0845 kJ/mol, the heat of vaporization of the water molecule H₂O is a high value of 40.8 kJ/mol (9.74666 kcal/mol). It is a known fact that such a high value is a result of the hydrogen bonding acting between water molecules H₂O. As described above, the water molecule H₂O is easily linked to a molecule having a polar group due to the strong polarity. That is, the water molecule H₂O is also easily linked to proteins having a polar group. This fact makes conjugation of tissues difficult in the presence of water molecules H₂O.

The reason that current treatment devices require energy for conjugation of tissues is none other than removal of water molecules H₂O. Removing water molecules H₂O present between tissues to be joined in conjugation of tissues can be said to be a condition for achieving stable and tight conjugation.

On the other hand, it is self-evident that a large quantity of fluid is present in a living body. In addition to fluid present in each tissue, a large quantity of fluid is also present outside tissues or outside organs such as various digestive juices, lubricants, and physiological saline given for treatment.

There is no doubt that fluid present inside and outside tissues is also present near tissues of a joint portion of body tissues whose conjugation has been completed. Water molecules H₂O present near a joint portion have, as described above, a strong polarity and are easily linked to polar groups present therearound. This is also no exception for polar groups whose linkage with other polar groups is once completed. The completed linkage is dissociated and a water molecule H₂O linked therein instead. Thus, the linkage of proteins is dissociated and the strength of conjugation between body tissues is weakened over time when viewed macroscopically.

### Disclosure of Invention

An object of the present invention is to provide a medical treatment device, a medical treatment system, and a medical treatment method capable of preventing invasion of fluid to a joint portion between body tissues to be able to sustain a state in which body tissues are in close contact for a long time, that is, sustaining the strength of the joint portion satisfactorily.

A medical treatment device configured to treat body tissues for conjugation according to the present invention includes at least a pair of holding members to hold the body tissues to be treated, an energy output portion provided in at least one of the pair of holding members and connected to an energy source to form a joined portion by supplying energy to the body tissues held by the pair of holding members and joining the body tissues, and a conjugation maintenance assistance portion capable of coating the body tissues to be treated with a substance capable of preventing fluid from invading so as to assist to maintain a joined state of the body tissues.

### Brief Description of Drawings

FIG. 1 is a schematic diagram showing a medical treatment system according to a first embodiment.
FIG. 2 is a schematic block diagram showing the medical treatment system according to the first embodiment.
FIG. 3A is a schematic longitudinal sectional view showing a closed treatment portion and a shaft of a bipolar type energy treatment device of the medical treatment system according to the first embodiment.
FIG. 3B is a schematic longitudinal sectional view showing the open treatment portion and the shaft of the energy treatment device of the medical treatment system according to the first embodiment.
FIG. 4A is a schematic plan view viewed from an arrow 4A direction in FIG. 4B, and shows a first holding member of the treatment portion of the energy treatment device of the medical treatment system according to the first embodiment.
FIG. 4B is a schematic longitudinal sectional view along a 4B-4B line in FIGS. 4A and 4C, and shows the first holding member of the treatment portion of the energy treatment device of the medical treatment system according to the first embodiment.
FIG. 4C is a schematic transverse sectional view along a 4C-4C line in FIG. 4A, and shows the first holding member of the treatment portion of the energy treatment device of the medical treatment system according to the first embodiment.
FIG. 5 is a schematic graph showing a relationship between the time and impedance when body tissues are held by the treatment portion of the energy treatment device of the medical treatment system according to the first embodiment and high-frequency energy is applied to the held body tissues.
FIG. 6A is a rough perspective view showing a mesh-shaped coating member disposed between body tissues when the body tissues are treated to join by the medical treatment system according to the first embodiment.
FIG. 6B is a rough perspective view showing a porous coating member disposed between body tissues when the body tissues are treated to join by the medical treatment system according to the first embodiment.
FIG. 6C is a rough transverse sectional view showing the state in which body tissues are treated to join by the medical treatment system while the body tissues to be joined are held by a treatment portion of an energy treatment device in a state in which the coating member is sandwiched between the body tissues to be joined according to the first embodiment.
FIG. 7 is a flow chart showing a state of control of the medical treatment system exercised by an energy source and a foot switch when body tissues are joined and an outer circumference of the joined body tissue is coated by using the medical treatment system according to the first embodiment.
FIG. 8 is a schematic graph showing the relationship between the time and a phase difference when body tissues are held by the treatment portion of the energy treatment device of the medical treatment system and the high-frequency energy is applied to the held body tissues according to a first modification of the first embodiment.
FIG. 9 is a schematic block diagram showing the medical treatment system when a change of the phase difference is used as a threshold of supplying the high-frequency energy/stopping the supply of the high-frequency energy for treatment according to the first modification of the first embodiment.
FIG. 10A is a schematic plan view viewed from an arrow 10A direction in FIG. 11B, and shows a first holding member of a treatment portion of an energy treatment device of a medical treatment system according to a second modification of the first embodiment.
FIG. 10B is a schematic transverse sectional view along a 10B-10B line in FIG. 10A, and shows the first holding member of the treatment portion of the energy treatment device of the medical treatment system according to the second modification of the first embodiment.
FIG. 11A is a rough perspective view showing the state in which a coating member is disposed on a main body of each of the first holding member and a second holding member of the treatment portion of the energy treatment device of the medical treatment system according to a third modification of the first embodiment.
FIG. 11B is a rough perspective view showing the coating member disposed on the main body of the first holding member and the second holding member of the treatment portion of the energy treatment device of the medical treatment system according to the third modification of the first embodiment.
FIG. 12A is a rough perspective view showing a sheet-shaped coating member disposed on the main body of the first and second holding members of the treatment portion of the energy treatment device of the medical treatment system according to the third modification of the first embodiment.
FIG. 12B is a rough perspective view showing a porous coating member disposed on the main body of the first and second holding members of the treatment portion of the energy treatment device of the medical treatment system according to the third modification of the first embodiment.
FIG. 12C is a rough perspective view showing a mesh-shaped coating member disposed on the main body of the first and second holding members of the treatment portion of the energy treatment device of the medical treatment system according to the third modification of the first embodiment.
FIG. 13 is a flow chart showing the state of control of the medical treatment system exercised by an energy source and a foot switch when body tissues are treated by using the medical treatment system according to the third modification of the first embodiment.
FIG. 14 is a rough perspective view showing the state in which the coating member is disposed on a surface of body tissues when the body tissues are treated by using the energy treatment device of the medical treatment system according to the third modification of the first embodiment.
FIG. 15A is a rough plan view viewed from an arrow 42A direction in FIGS. 15B and 15C, and shows a first holding member of a treatment portion of an energy treatment device of a medical treatment system according to a fourth modification of the first embodiment.
FIG. 15B is a rough longitudinal sectional view along a 15B-15B line in FIGS. 15A and 15C, and shows the first holding member of the treatment portion of the energy treatment device of the medical treatment system according to the fourth modification of the first embodiment.
FIG. 15C is a schematic transverse sectional view along a 15C-15C line in FIGS. 15A and 15B, and shows the first holding member of the treatment portion of the energy treatment device of the medical treatment system according to the fourth modification of the first embodiment.
FIG. 16 is a schematic diagram showing the state of body tissues being treated by using the monopolar type energy treatment device of the medical treatment system according to a fifth modification of the first embodiment.
FIG. 17 is a schematic diagram showing the medical treatment system according to a sixth modification of the first embodiment.
FIG. 18 is a schematic diagram showing a medical treatment system according to a second embodiment.
FIG. 19 is a schematic block diagram showing the medical treatment system according to the second embodiment.
FIG. 20A is a schematic plan view viewed from an arrow 20A direction in FIGS. 20B and 20C, and shows a first holding member of the treatment portion of the energy treatment device of the medical treatment system according to the second embodiment.
FIG. 20B is a schematic longitudinal sectional view along a 20B-20B line in FIGS. 20A and 20C, and shows the first holding member of the treatment portion of the energy treatment device of the medical treatment system according to the second embodiment.
FIG. 20C is a schematic transverse sectional view along a 20C-20C line in FIGS. 20A and 20B, and shows the first holding member of the treatment portion of the energy treatment device of the medical treatment system according to the second embodiment.
FIG. 21 is a flow chart showing a state of control of the medical treatment system exercised by an energy source, a foot switch, and a fluid source when body tissues are joined and an outer circumference of the joined body tissue is coated by using the medical treatment system according to the second embodiment.
FIG. 22A is a schematic plan view viewed from an arrow 22A direction in FIGS. 22B and 22C, and shows a first holding member of the treatment portion of the energy treatment device of the medical treatment system according to a first modification of the second embodiment.
FIG. 22B is a schematic longitudinal sectional view along a 22B-22B line in FIGS. 22A and 22C, and shows the first holding member of the treatment portion of the energy treatment device of the medical treatment system according to the first modification of the second embodiment.
FIG. 22C is a schematic transverse sectional view along a 22C-22C line in FIGS. 22A and 22B, and shows the first holding member of the treatment portion of the energy treatment device of the medical treatment system according to the first modification of the

### second embodiment.

FIG. 22D is a rough perspective view showing a projection disposed on a high-frequency electrode of the first holding member of the treatment portion of the energy treatment device of the medical treatment system according to the first modification of the second embodiment.
FIG. 23A is a rough plan view viewed from an arrow 23A direction in FIGS. 23B and 23C, and shows a second holding member of the treatment portion of the energy treatment device of the medical treatment system according to the first modification of the second embodiment.
FIG. 23B is a rough longitudinal sectional view along a 23B-23B line in FIGS. 23A and 23C, and shows the second holding member of the treatment portion of the energy treatment device of the medical treatment system according to the first modification of the second embodiment.
FIG. 23C is a rough transverse sectional view along a 23C-23C line in FIGS. 23A and 23B, and shows the second holding member of the treatment portion of the energy treatment device of the medical treatment system according to the first modification of the second embodiment.
FIG. 24 is a rough perspective view showing the state of body tissues immediately after being treated by using the energy treatment device of the medical treatment system according to the first modification of the second embodiment.
FIG. 24A is a flow chart showing a control state of the medical treatment system when body tissues are joined by supplying energy from the energy source after an adhesive is applied to contact surfaces of the body tissues before the body tissues being joined by using the medical treatment device according to the first modification of the second embodiment.
FIG. 25 is a schematic diagram showing a medical treatment system according to a third embodiment.
FIG. 26 is a schematic block diagram showing the medical treatment system according to the third embodiment.
FIG. 27A is a schematic plan view viewed from an arrow 27A direction in FIGS. 27B and 27C, and shows a first holding member of the treatment portion of the energy treatment device of the medical treatment system according to the third embodiment.
FIG. 27B is a schematic longitudinal sectional view along a 27B-27B line in FIGS. 27A and 27C, and shows the first holding member of the treatment portion of the energy treatment device of the medical treatment system according to the third embodiment.
FIG. 27C is a schematic transverse sectional view along a 27C-27C line in FIGS. 27A and 27B, and shows the first holding member of the treatment portion of the energy treatment device of the medical treatment system according to the third embodiment.
FIG. 28A is a schematic longitudinal sectional view showing the closed treatment portion and a shaft of the bipolar type energy treatment device of the medical treatment system according to the third embodiment.
FIG. 28B is a schematic longitudinal sectional view showing the open treatment portion and the shaft of the energy treatment device of the medical treatment system according to the third embodiment.
FIG. 29A is a rough perspective view showing a tip portion containing a cutting portion of a cutter disposed on the energy treatment device of the medical treatment system according to the third embodiment.
FIG. 29B is a rough transverse sectional view showing the cutter disposed on the energy treatment device of the medical treatment system according to the third embodiment.
FIG. 29C is a rough transverse sectional view showing the state of treating and conjugating body tissues while being held by the treatment portion of the energy treatment device of the medical treatment system and cutting the body tissues by the cutter according to the third embodiment.
FIG. 29D is a rough perspective view showing the state of body tissues immediately after being treated by using the energy treatment device of the medical treatment system according to the third embodiment.
FIG. 30 is a flow chart showing the state of control of the medical treatment system exercised by an energy source, a foot switch, and a fluid source when body tissues are treated by using the medical treatment system according to the third embodiment.
FIG. 31A is a rough perspective view showing a tip portion containing a cutting portion of a cutter disposed on an energy treatment device of a medical treatment system according to a first modification of the third embodiment.
FIG. 31B is a rough transverse sectional view showing the cutter disposed on the energy treatment device of the medical treatment system according to the first modification of the third embodiment.
FIG. 31C is a rough transverse sectional view showing the state of treating and conjugating body tissues while being held by the treatment portion of the energy treatment device of the medical treatment system and being cut by the cutter according to the first modification of the third embodiment.
FIG. 31D is a rough perspective view showing the state of body tissues immediately after being treated by using the energy treatment device of the medical treatment system according to the first modification of the third embodiment.
FIG. 32A is a rough plan view viewed from an arrow 32A direction in FIG. 32B, and shows a first holding member of a treatment portion of an energy treatment device of a medical treatment system according to a second modification of the third embodiment.
FIG. 32B is a rough transverse sectional view along a 32B-32B line in FIG. 32A, and shows the first holding member of the treatment portion of the energy treatment device of the medical treatment system according to the second modification of the third embodiment.
FIG. 33 is a flow chart showing the state of control of the medical treatment system exercised by an energy source, a foot switch, and a fluid source when body tissues are treated by using the medical treatment system according to the second modification of the third embodiment.
FIG. 34 is a rough perspective view showing the state in which the coating member is disposed on a surface of body tissues when the body tissues are treated by using the energy treatment device of the medical treatment system according to the second modification of the third embodiment.
FIG. 35 is a flow chart showing the state of control of the medical treatment system exercised by an energy source, a foot switch, and a fluid source when body tissues are treated by using the medical treatment system according to the second modification of the third embodiment.
FIG. 36 is a flow chart showing the state of control of the medical treatment system exercised by an energy source, a foot switch, and a fluid source when body tissues are treated by using the medical treatment system according to the second modification of the third embodiment.
FIG. 37A is a schematic diagram showing a medical treatment system according to a fourth embodiment.
FIG. 37B is a rough partial longitudinal sectional view showing a handle of an energy treatment device of the medical treatment system according to the fourth embodiment.
FIG. 38 is a rough block diagram showing the medical treatment system according to the fourth embodiment.
FIG. 39A is a rough longitudinal sectional view showing a closed treatment portion and a shaft of the energy treatment device of the bipolar type of the medical treatment system according to the fourth embodiment.
FIG. 39B is a rough longitudinal sectional view showing the open treatment portion and the shaft of the energy treatment device of the medical treatment system according to the fourth embodiment.
FIG. 40A is a rough plan view showing a first holding member of the treatment portion of the energy treatment device of the medical treatment system according to the fourth embodiment.
FIG. 40B is a rough transverse sectional view along a 40B-40B line in FIG. 39A showing the state in which body tissues are held by the treatment portion of the energy treatment device of the medical treatment system according to the fourth embodiment.
FIG. 41 is a rough partial longitudinal sectional view showing a modification of the handle of the energy treatment device of the medical treatment system according to the fourth embodiment.
FIG. 42 is a schematic diagram showing a medical treatment system according to a fifth embodiment.
FIG. 43A is a rough front view showing the state in which a main body-side holding member and a detachable-side holding member of a treatment portion of a bipolar type energy treatment device of the medical treatment system are detached according to the fifth embodiment.
FIG. 43B is a rough longitudinal sectional view along a 43B-43B line in FIG. 43A, and shows the state in which the main body-side holding member and the detachable-side holding member of the treatment portion of the energy treatment device of the medical treatment system are detached according to the fifth embodiment.
FIG. 44 is a rough plan view viewed from an arrow 44 direction in FIG. 43B, and shows the main body-side holding member of the treatment portion of the energy treatment device of the medical treatment system according to the fifth embodiment.
FIG. 45A is a rough front view showing the state in which the main body-side holding member and the detachable-side holding member of the treatment portion of the bipolar type energy treatment device of the medical treatment system are closed according to the fifth embodiment.
FIG. 45B is a rough longitudinal sectional view showing the state in which the main body-side holding member and the detachable-side holding member of the treatment portion of the bipolar type energy treatment device of the medical treatment system are open according to the fifth embodiment.
FIG. 45C is a rough perspective view showing a projection disposed on a high-frequency electrode of the detachable-side holding member of the treatment portion of the energy treatment device of the medical treatment system according to the fifth embodiment.

### Best Mode for Carrying Out the Invention

The best mode for carrying out the present invention will be described below with reference to drawings.

### [First embodiment]

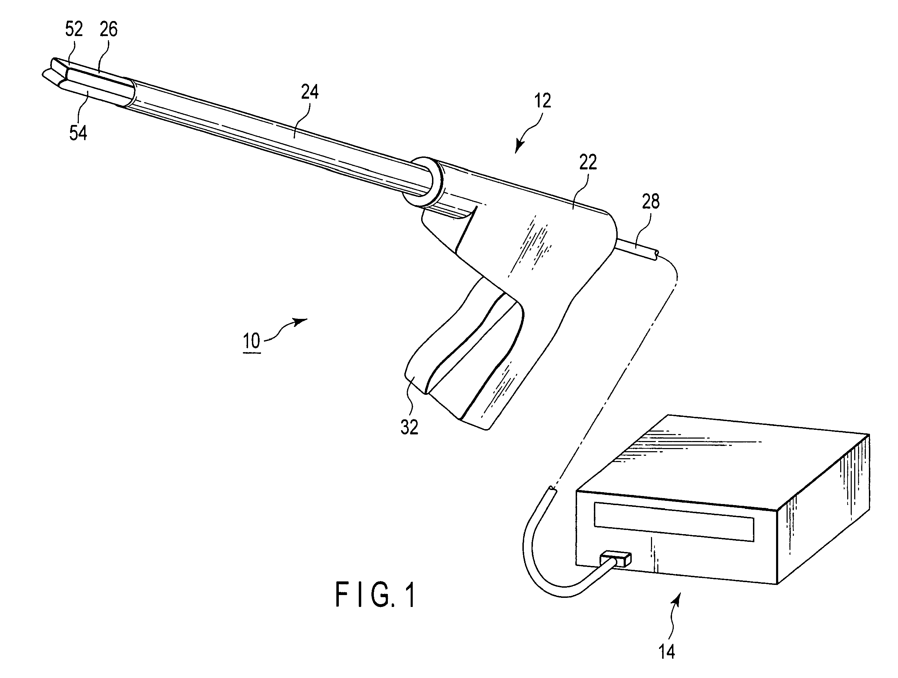

The first embodiment will be described with reference to FIGS. 1 to 7.

For example, a linear-type surgical treatment device 12 for treatment through the abdominal wall is taken as an example of the energy treatment device (medical treatment device).

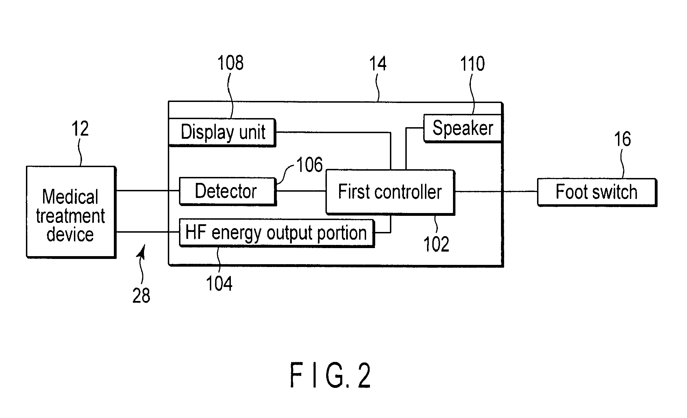

As shown in FIGS. 1 and 2, a medical treatment system 10 includes the energy treatment device 12, an energy source (control section) 14, and a foot switch (or a hand switch) 16.

As shown in FIG. 1, the energy treatment device 12 includes a handle 22, a shaft 24, and a treatment portion (holding portion) 26 which is able to be opened and closed. The handle 22 is connected to the energy source 14 via a cable 28. As shown in FIG. 2, the foot switch 16 is connected to the energy source 14.

The foot switch 16 includes a pedal (not shown). A series of operations such as ON/OFF of the supply of energy (high-frequency energy in the present embodiment) from the energy source 14 to the surgical treatment device 12 can be switched by the pedal of the foot switch 16 being operated (pressed/released) by an operator. While the pedal is pressed, high-frequency energy is output based on an appropriately set state (state in which the output quantity of energy, timing of energy output and the like are controlled). When pedal pressing is released, the output of high-frequency energy is forced to stop.

As shown in FIG. 1, the handle 22 is formed in a shape that makes it easier for the operator to grip and is formed, for example, in a substantially L shape. The shaft 24 is disposed at one end of the handle 22. The cable 28 described above is extended from a proximal end of the handle 22 which is coaxial with the shaft 24. Electrical connection lines 28a, 28b of high-frequency electrodes 92, 94 described later are inserted into the cable 28.

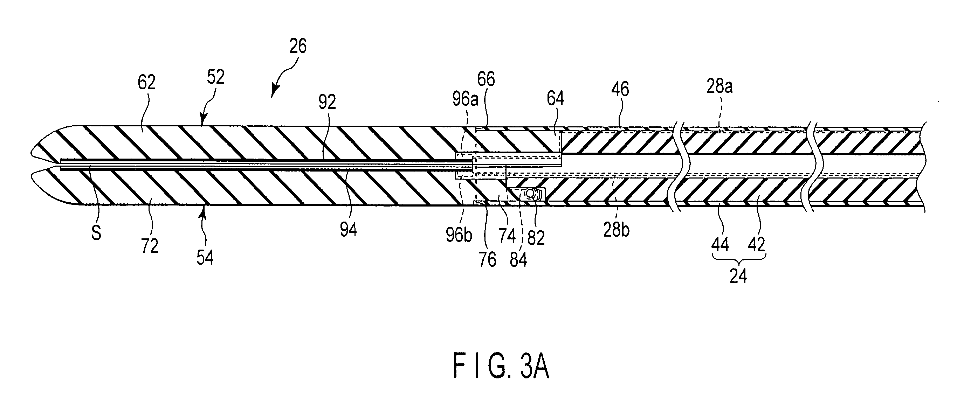

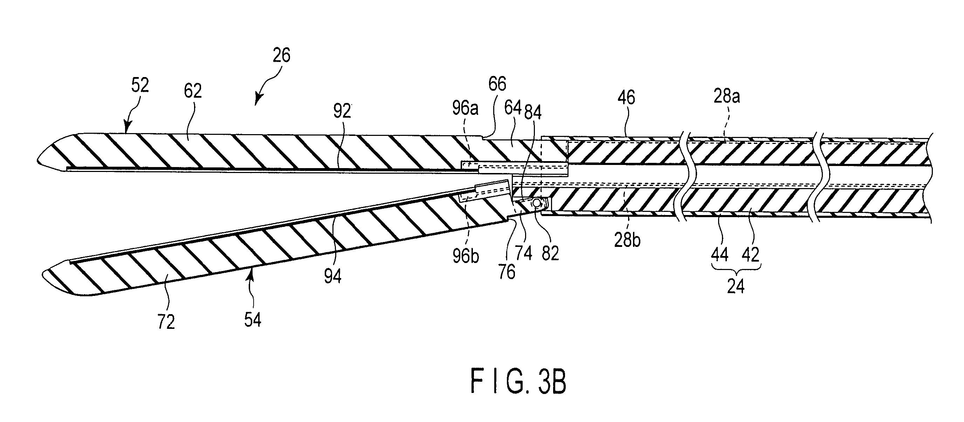

On the other hand, the other end side of the handle 22 is a gripper extending in a direction away from an axial direction of the shaft 24 and gripped by the operator. The handle 22 includes a treatment portion opening/closing knob 32 being arranged side by side. The treatment portion opening/closing knob 32 is coupled to the proximal end of a sheath 44 (see FIGS. 3A and 3B) described later of the shaft 24 in a substantially center portion of the handle 22. If the treatment portion opening/closing knob 32 is moved closer to or away from the other end of the handle 22, the sheath 44 moves along the axial direction thereof.

As shown in FIGS. 3A and 3B, the shaft 24 includes a pipe 42 and the sheath 44 slidably disposed on the outer side of the pipe 42. The base end of the pipe 42 is fixed to the handle 22 (see FIG. 1). The sheath 44 is slidable along the axial direction of the pipe 42.

A recess 46 is formed on the outer side of the pipe 42 along the axial direction thereof. An electrode connection line 28a connected to the high-frequency electrode (energy output portion) 92 described later is disposed in the recess 46. An electrode connection line 28b connected to the high-frequency electrode (energy output portion) 94 described later is inserted into the pipe 42.

As shown in FIG. 1, the treatment portion 26 is disposed at the tip of the shaft 24. As shown in FIGS. 3A and 3B, the treatment portion 26 includes a pair of holding members 52, 54, that is, the first holding member (first jaw) 52 and the second holding member (second jaw) 54.

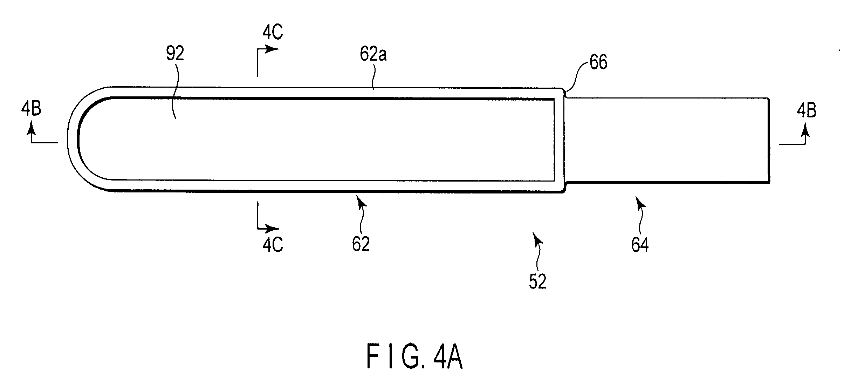

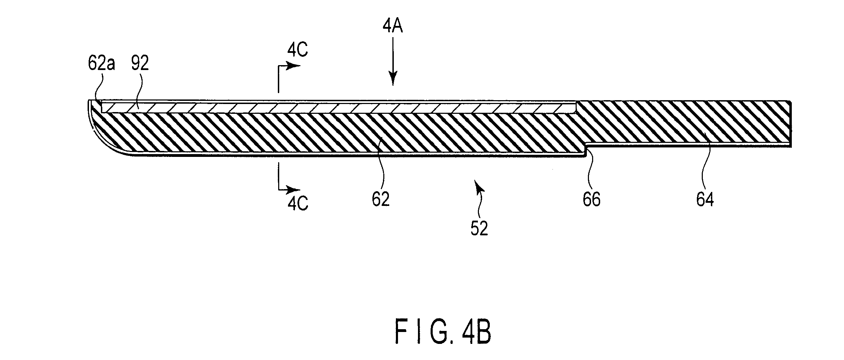

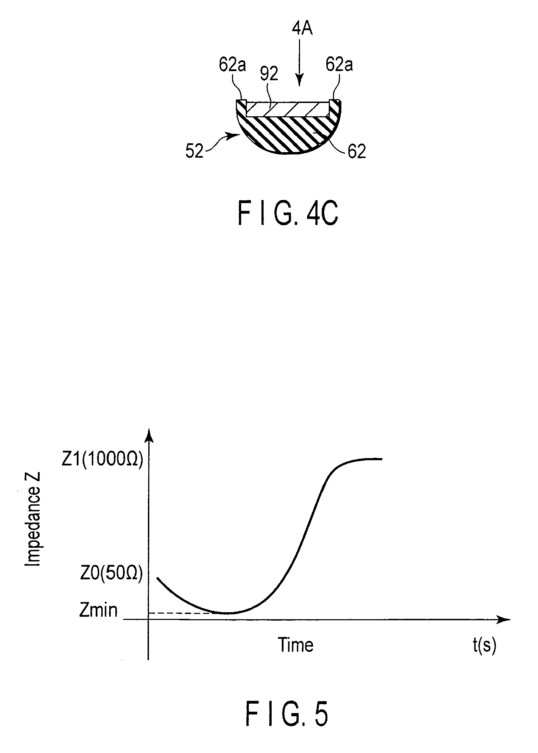

The first and second holding members 52, 54 shown in FIGS. 3A and 3B each have suitably insulating properties as a whole. As shown in FIGS. 4A to 4C, the first holding member 52 integrally includes a first holding member main body (hereinafter, referred to mainly as a main body) 62 and a base 64 provided in the proximal end of the main body 62. The main body 62 is a portion which holds body tissues L1, L2 shown in FIG. 5B in collaboration with a main body 72 described later of the second holding member 54 and has a holding surface (edge) 62a. The base 64 is a portion coupled to the tip of the shaft 24. The main body 62 and the base 64 of the first holding member 52 are disposed coaxially. Then, a step 66 is formed between the main body 62 and the base 64.

The second holding member 54 integrally includes, though not illustrated in detail like the first holding member 52 shown in FIGS. 4A to 4C, a second holding member body (hereinafter, referred to mainly as a main body) 72 and a base 74 provided in the proximal end of the main body 72. The main body 72 is a portion that holds the body tissues L1, L2 in collaboration with the main body 62 of the first holding member 52 and has a holding surface (edge) 72a. The base 74 is a portion coupled to the tip of the shaft 24. The main body 72 and the base 74 of the second holding member 54 are disposed coaxially. Then, a step 76 is formed between the main body 72 and the base 74.

In the present embodiment and embodiments described below, the main body 62 of the first holding member 52 and the main body 72 of the second holding member 54 have the same shape. Though the base 74 of the second holding member 54 is different from the base 64 of the first holding member 52 in that the base 74 of the second holding member 54 is formed, as will be described later, so as to be pivotally supported by the pipe 42 of the shaft 24, the base 64 of the first holding member 52 and the base 74 of the second holding member 54 have the same structure in other respects and thus, the description thereof is omitted when appropriate.

As shown in FIG. 4C, an exterior surface of the main body 62 of the first holding member 52 is formed as a smooth curved surface. Though not shown, the exterior surface of the base 64 of the first holding member 52 is also formed as a smooth curved surface. In a state in which the second holding member 54 is closed with respect to the first holding member 52, the transverse section of the treatment portion 26 is formed in a substantially circular shape or a substantially elliptic shape along with the transverse sections of the main bodies 62, 72 and the bases 64, 74. In a state in which the second holding member 54 is closed with respect to the first holding member 52, the holding surfaces (edges) 62a, 72a of the main bodies 62, 72 of the first and second holding members 52, 54 are mutually opposite to each other and in contact. Incidentally, in this state, the outside diameter of the base end of the main bodies 62, 72 of the first and second holding members 52, 54 is formed larger than the outside diameter of the bases 64, 74. Then, the steps 66, 76 described above are formed between the main bodies 62, 72 and the bases 64, 74, respectively.

The first holding member 52 has the base 64 thereof fixed to the tip portion of the pipe 42 of the shaft 24. On the other hand, the second holding member 54 has the base 74 thereof rotatably supported on the tip portion of the pipe 42 of the shaft 24 by a support pin 82 disposed in a direction perpendicular to the axial direction of the shaft 24. The second holding member 54 can be opened and closed with respect to the first holding member 52 by being rotated around the axis of the support pin 82. The second holding member 54 is energized by, for example, an elastic member 84 such as a plate spring so as to be opened with respect to the first holding member 52.

The first and second holding members 52, 54 are formed in a closed state of the second holding member 54 with respect to the first holding member 52 in such a way that an outer circumferential surface in a substantially circular shape or a substantially elliptic shape together with the bases 64, 74 thereof is substantially flush with the outer circumferential surface of the tip portion of the pipe 42 or slightly larger. Thus, the sheath 44 can be slid with respect to the pipe 42 so as to cover the bases 64, 74 of the first and second holding members 52, 54 with the tip of the sheath 44.

In this state, as shown in FIG. 3A, the second holding member 54 is closed with respect to the first holding member 52 against an energizing force of the elastic member 84. On the other hand, if the sheath 44 is slid to the proximal end side of the pipe 42 from the state in which the bases 64, 74 of the first and second holding members 52, 54 are covered with the tip of the sheath 44, as shown in FIG. 3B, the second holding member 54 is opened with respect to the first holding member 52 due to an energizing force of the elastic member 84.

The plate-like high-frequency electrodes (joining members) 92, 94 are disposed as an output member and an energy discharge portion inside the holding surfaces (edges) 62a, 72a of the main bodies 62, 72 of the first and second holding members 52, 54. These high-frequency electrodes 92, 94 are electrically connected to the tip of the electrical connection lines 28a, 28b via connectors 96a, 96b. Then, these electrical connection lines 28a, 28b are connected to a high-frequency energy output portion 104 described later of the energy source 14. Thus, the body tissues L1, L2 are heated and denatured by passing power through the body tissues L1, L2 held between the high-frequency electrodes 92, 94 to generate Joule heat in the body tissues L1, L2.

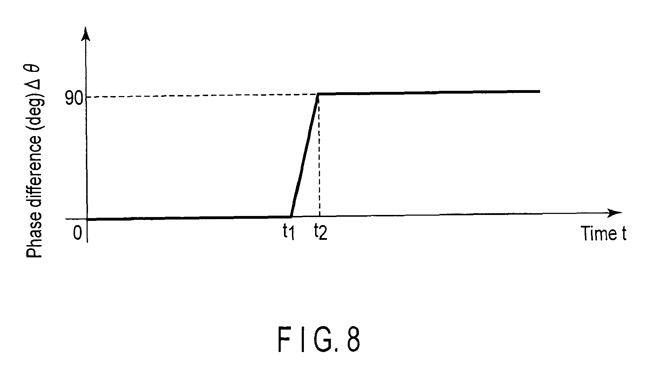

These high-frequency electrodes 92, 94 can be used, in addition to treatment of the body tissues L1, L2 by high-frequency energy, as a sensor to measure an impedance Z (see FIG. 5) between the body tissues L1, L2 or a phase θ (see FIG. 8). The high-frequency electrodes 92, 94 can transmit/receive a signal to/from a detector 106 described later of the energy source 14 through, for example, the electrical connection lines 28a, 28b. It is assumed here that the impedance Z is measured by the detector 106.

As shown in FIG. 2, the energy source 14 includes a first controller (energy control unit) 102, the high-frequency energy output portion (first high-frequency energy output unit) 104, the detector 106, a display unit 108, and a speaker 110. The high-frequency energy output portion 104, the detector 106, the display unit 108, and the speaker 110 are connected to the first controller 102 so that the high-frequency energy output portion 104, the detector 106, the display unit 108, and the speaker 110 are controlled by the first controller 102.

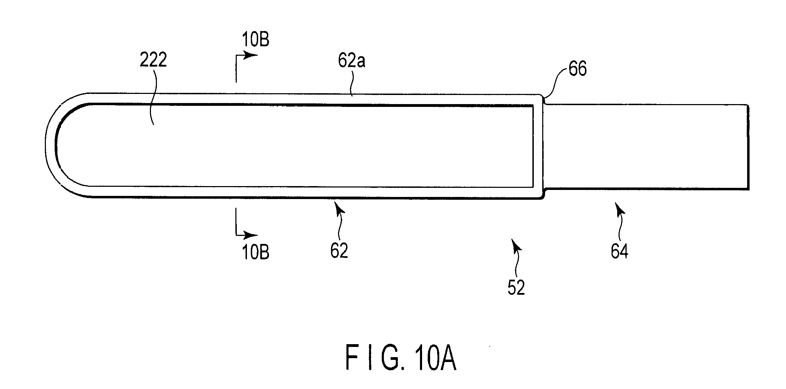

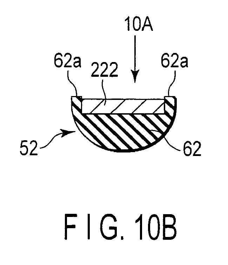

The high-frequency energy output portion 104 generates energy and supplies the energy to the high-frequency electrodes 92, 94 via the electrical connection lines 28a, 28b. Incidentally, the high-frequency energy output portion 104 also functions as an energy output portion that supplies energy to heaters 222, 232 (see FIGS. 10A and 10B) that will be described in the second modification.

The detector 106 detects measurement results obtained by the high-frequency electrodes 92, 94 holding the body tissues L1, L2 through the electrical connection lines 28a, 28b to calculate the impedance Z. The display unit 108 is a unit in which various settings are made such as the setting of a threshold Z1 of the impedance Z while a setting is checked through the display. The speaker 110 has a sound source (not shown) and produces a sound when a treatment is finished or a problem arises. The sound used to tell the end of treatment and the sound used to tell an occurrence of problem have different tones. The speaker 110 can also produce a distinct sound during treatments, for example, a sound to tell the end of the first step of the treatment and a sound to tell the end of the second step of the treatment.

The foot switch 16 is connected to the first controller 102 of the energy source 14. Thus, if the foot switch 16 is operated, the energy source 14 works.

If the foot switch 16 is changed to ON (a pedal not shown is pressed), a treatment by the energy treatment device 12 is carried out and if the foot switch 16 is changed to OFF (the pedal is released), the treatment stops. The display unit 108 functions as a setting unit (controller) when an output quantity (the output quantity itself or what kind of treatment to adopt (treatment for the purpose of joining the body tissues L1, L2, treatment for the purpose of sealing openings of the body tissues or the like)) of the high-frequency energy output portion 104 or output timing of energy is controlled by the first controller 102. It is needless to say that the display unit 108 has a display function to display what is set.

The detector 106 can detect (calculate) the impedance Z of the body tissues L1, L2 between the first and second high-frequency electrodes 92, 94 through the first and second high-frequency electrodes 92, 94 that output high-frequency energy. That is, the detector 106 and the first and second high-frequency electrodes 92, 94 have a sensor function to measure the impedance Z of the body tissues L1, L2 between the first and second high-frequency electrodes 92, 94.

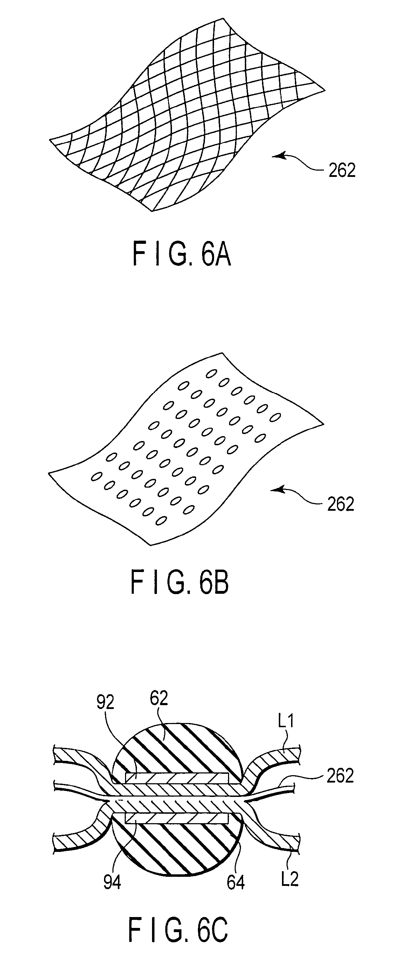

In the present embodiment, a conjugation assistance member 262 shown in FIGS. 6A and 6B is disposed between the body tissues L1, L2 to be joined. The conjugation assistance member 262 is a substance (conjugation adjunct) capable of preventing fluid from invading into a body tissue L_{T} and is formed in a mesh (see FIG. 6A) shape, porous shape (see FIG. 6B) or other shapes. The substance capable of preventing fluid from invading into the body tissue L_{T} is preferably a bioabsorbable material absorbed after being invaded into body tissues when applied to the body tissues. The conjugation assistance member 262 has a mesh or porous shape in order to be able to pass a current between the electrodes 92, 94 by bringing the body tissues L1, L2 to be joined into contact. The conjugation assistance member 262 in a non-porous shape can be used similarly by forming a hole by an appropriate portion.

The substance (join condition sustainment assistance portion 262) which prevents fluid from invading the body tissue L_{T} contains a compound. The compound is the substance which is configured to coat or join the body tissues L1, L2 by a physical action, a chemical action, or both actions. The compound preferably contains at least one of protein, glucide, polymer, and hardener. The protein suitably contains at least one of fibrin, albumin, collagen, and gelatin. The glucide suitably contains at least one of starch, hyaluronic acid, and chitosan. The polymer is suitably polyethylene glycol, polyglycolic acid, polylactic acid, or polycaprolactam. The hardener is suitably an acrylate derivative, aldehyde derivative, succinimide derivative, or isocyanate derivative. That is, for example, an organic adhesive, inorganic adhesive, bonding biomaterial, crosslinking agent, and monomer/polymer resins can be cited as a substance (joining adjunct) to prevent fluid from penetrating body tissues. Further, for example, the join condition sustainment assistance portion 262 may contain an antibiotic, growth promoter and the like.

Table 1 shows main components of eight auxiliary joining members used for experiments to join the body tissues L1, L2 described below and corresponding types of the auxiliary joining members. It is needless to say that main components and types of the auxiliary joining members are not limited to the main components and types shown in Table 1.

**Table 1 Main components and types of the auxiliary joining members used for experiments to join body tissues**

| No. | Main component | Type |
|---|---|---|
| (1) | Cyanoacrylate monomer | Cyanoacrylate adhesive |
| (2) | Fibrinogen | Fibrin adhesive |
| | Thrombin | |
| (3) | Glutaraldehyde (crosslinking agent) | Aldehyde adhesive |
| | Albumin (main agent) | |
| (4) | Formaldehyde (crosslinking agent) | |
| | Glutaraldehyde (crosslinking agent) | |
| | Gelatin (main agent) | |
| (5) | Organic succinimide (crosslinking agent) | Succinimide adhesive |
| | Albumin (main agent) | |
| (6) | PEG succinimide (crosslinking agent) | |
| | Albumin (main agent) | |
| (7) | Polyglycolic acid | Biodegrative polymer |
| (8) | Polycaprolactam | Biodegrative polymer |

Then, when heated to an appropriate temperature, the heated portion of the conjugation assistance member 262 is melted and components of the conjugation adjunct spread to the surface of body tissues and invade and are cured while spread on the surface of body tissues and invaded when cooled. When the conjugation assistance member 262 is cured, the action of preventing fluid from invading to contact surfaces described later or the like from outside body tissues is achieved.

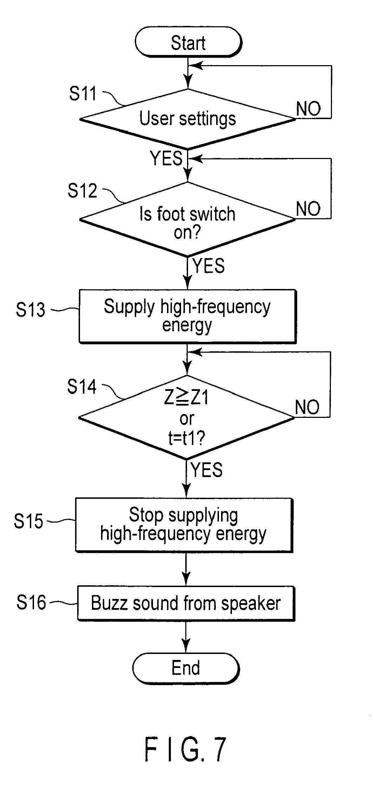

FIG. 5 shows a relationship between an energy supply time t of the body tissues L1, L2 between the high-frequency electrodes 92, 94 and the impedance Z between the body tissues L1, L2 when desired energy is supplied from the high-frequency energy output portion 104 to the high-frequency electrodes 92, 94 and high-frequency treatment of the body tissues L1, L2 is carried out. FIG. 7 shows an example of the control flow of the surgical treatment device 12 by the high-frequency energy output portion 104.

Next, the action of the medical treatment system 10 according to the present embodiment will be described.

The operator operates the display unit 108 of the energy source 14 in advance to set output conditions for the medical treatment system 10 (step S11). The operator checks the output (set power Pset [W]) from the high-frequency energy output portion 104, the threshold Z1 [Ω] of the impedance Z by the detector 106, a maximum energy supply time t1 [sec] and the like through the display unit 108. If the output from the high-frequency energy output portion 104 or the threshold Z1 of the impedance Z by the detector 106 should be set to a different value, the operator sets the value as desired and checks the value through the display unit 108.

As shown in FIG. 3A, the treatment portion 26 and the shaft 24 of the surgical treatment device 12 are inserted into the abdominal cavity through, for example, the abdominal wall in the state in which the second holding member 54 is closed to the first holding member 52. The treatment portion 26 of the surgical treatment device 12 is opposed to the body tissues L1, L2 to be treated (to be held). Then, the conjugation assistance member 262 is disposed between the body tissues L1, L2 by using, for example, forceps.

The operator operates the treatment portion opening/closing knob 32 of the handle 22 to hold the body tissues L1, L2 to be treated by the first holding member 52 and the second holding member 54. With this operation, the sheath 44 is moved to the side of the proximal end of the shaft 24 with respect to the pipe 42. The space between the bases 64, 74 can no longer be sustained in a cylindrical shape due to the energizing force of the elastic member 84 and the second holding member 54 is opened with respect to the first holding member 52.

The body tissues L1, L2 to be joined (to be treated) are arranged between the high-frequency electrodes 92, 94 of the first and second holding members 52, 54. The treatment portion opening/closing knob 32 of the handle 22 is operated in this state. In this case, the sheath 44 is moved to the distal side of the shaft 24 with respect to the pipe 42. The space between the bases 64, 74 is closed by the sheath 44 against the energizing force of the elastic member 84 and to make it into a cylindrical shape. Thus, the main body 62 of the first holding member 52 formed integrally with the base 64 and the main body 72 of the second holding member 54 formed integrally with the base 74 are closed. That is, the second holding member 54 is closed with respect to the first holding member 52. In this manner, the body tissues L1, L2 to be joined are held between the first holding member 52 and the second holding member 54.

Since the auxiliary joining member 262 is disposed between the body tissues L1, L2, the auxiliary joining member 262 is held between the body tissues L1, L2 by holding the body tissues L1, L2 by the first and second holding members 52, 54.

At this point, the body tissue L1 to be treated is in contact with the high-frequency electrode 92 of the first holding member 52 and the body tissue L2 to be treated is in contact with the high-frequency electrode 94 of the second holding member 54. Peripheral tissues of the body tissues L1, L2 to be joined are in close contact with both contact surfaces opposite to the holding surface (edge) 62a of the main body 62 of the first holding member 52 and the holding surface (edge) 72a of the main body 72 of the second holding member 54. A contact surface C1 of the body tissue L1 is in contact with a contact surface C2 of the body tissue L2 in such a manner that pressure is applied to each other.

Thus, the operator operates the pedal of the foot switch 16 while the body tissues L1, L2 are held between the first holding member 52 and the second holding member 54. A signal is input into the first controller 102 from the foot switch 16 and the first controller 102 of the energy source 14 determines whether the switch 16 is changed to ON by pressing the pedal thereof through the operation of the operator (S12).

If the first controller 102 determines that the switch 16 is changed to ON by pressing the pedal thereof, a signal is input into the high-frequency energy output portion 104 from the first controller 102. The high-frequency energy output portion 104 generates energy and supplies the energy to the body tissues L1, L2 between the high-frequency electrodes 92, 94 through the electrical connection lines 28a, 28b (S13). At this point, the high-frequency energy output portion 104 supplies the set power Pset [W] set in advance through the display unit 108, for example, power of about 20 [W] to 80 [W] to between the high-frequency electrode 92 of the first holding member 52 and the high-frequency electrode 94 of the second holding member 54.

Thus, the high-frequency energy output portion 104 passes a high-frequency current to the body tissues L1, L2 to be joined between the high-frequency electrode 92 of the first holding member 52 and the high-frequency electrode 94 of the second holding member 54. The auxiliary joining member 262 is formed in a mesh shape (FIG. 6A) or porous state (FIG. 6B) and thus, portions of contact surfaces C1, C2 of the body tissues L1, L2 are mutually in contact. That is, the high-frequency energy output portion 104 applies high-frequency energy to the body tissues L1, L2 held between the high-frequency electrodes 92, 94. Thus, the body tissues L1, L2 are heated by generating Joule heat in the body tissues L1, L2 held between the high-frequency electrodes 92, 94. Cell membranes inside the body tissues L1, L2 held between the high-frequency electrodes 92, 94 are destroyed by the action of Joule heat to release substances inside the cell membrane so that the substances are equalized with components outside the cell membrane including collagen. Since a high-frequency current is being passed to the body tissues L1, L2 between the high-frequency electrodes 92, 94, further Joule heat is acted on the equalized body tissues L1, L2 to conjugate, for example, the contact surfaces C1, C2 of the body tissues L1, L2 or layers of tissues. Therefore, if a high-frequency current is passed to the body tissues L1, L2 between the high-frequency electrodes 92, 94, the body tissues L1, L2 are heated and so the inside of the body tissues L1, L2 is denatured (the body tissues L1, L2 are burned) while the body tissues L1, L2 are dehydrated, generating a joined portion C after the contact surfaces C1, C2 are brought into close contact. In this manner, the two body tissues L1, L2 are joined to form the body tissue L_{T} having the joined portion C.

Then, the conjugation assistance member 262 is melted by the body tissues L1, L2 being heated, leading to the same condition as the substance capable of preventing fluid from invading into the body tissue L_{T} being applied to the whole contact surfaces C1, C2. Further, the substance capable of preventing fluid from invading into the body tissue L_{T} invading from the contact surfaces C1, C2 toward an exterior surface Sc in contact with the high-frequency electrodes 92, 94. Thus, the substance capable of preventing fluid from invading into the body tissue L_{T} infiltrates not only to the contact surfaces of the body tissues L1, L2, but also to tissues surrounding the contact surfaces. Therefore, the joined portion C of the body tissues L1, L2 can be formed in a wider range, as well as the contact surfaces C1, C2. That is, the joined portion C includes, in addition to the joined surfaces, surrounding tissues thereof. The substance capable of preventing fluid from infiltrating into the body tissue L_{T} is cured while invaded in the body tissue L_{T}.

With an increasing level of denaturation of the body tissues L1, L2, a fluid (for example, a liquid (blood) and/or a gas (vapor)) is released from the body tissues L1, L2. In this case, the holding surfaces 62a, 72a of the main bodies 62, 72 of the first and second holding members 52, 54 have higher adhesiveness to the body tissues L1, L2 than the high-frequency electrodes 92, 94. Thus, the holding surfaces 62a, 72a function as a barrier portion (dam) that inhibits a fluid from the body tissues L1, L2 from escaping to the outside of the first holding member 52 and the second holding member 54. That is, a thermal spread can be prevented from being generated in body tissues other than the body tissues L1, L2 to be treated and joined.

In this case, the high-frequency electrodes 92, 94 of the first and second holding members 52, 54 have a sensor function and thus transmit information (impedance Z) about between the held body tissues L1, L2 to the detector 106 through the electrical connection lines 28a, 28b. As shown in FIG. 5A, an initial value Z0 of the impedance Z when treatment is started (when the supply of high-frequency energy to between the body tissues L1, L2 is started) is, for example, about 50 [Ω] to 60 [Ω]. As the body tissues L1, L2 are increasingly burned by the high-frequency current flowing into the body tissues L1, L2, the impedance Z drops to Zmin (for example, about 10 [Ω]) and then gradually rises.

The first controller 102 controls the detector 106 so that information about the body tissues L1, L2 between the high-frequency electrodes 92, 94 is calculated at equal time intervals (for example, a few milliseconds). The first controller 102 determines whether the impedance Z during high-frequency energy output operated based on a signal from the detector 106 is equal to or more than the threshold Z1 (here, as shown in FIG. 5, about 1000 [Ω]) set (S11) in advance through the display unit 108 (S14). It is, needless to say, that the threshold Z1 of the impedance Z can appropriately be set.

The threshold Z1 is preferably, for example, larger than the initial value Z0 and in a position where the rate of rise the impedance Z value slows down

(see FIG. 5). If it is determined that the impedance Z has reached the threshold Z1 or is larger than the threshold Z1, a signal is transmitted from the first controller 102 to the high-frequency energy output portion 104. Then, the output from the high-frequency energy output portion 104 to the high-frequency electrodes 92, 94 of the first and second holding members 52, 54 is made to stop (S15).

On the other hand, if the impedance Z has not reached the threshold Z1, energy output will continue. If the impedance Z between the body tissues L1, L2 is determined to be smaller than the threshold Z1, high-frequency energy will continue to be given to the body tissues L1, L2 held between the high-frequency electrodes 92, 94 of the first and second holding members 52, 54. Then, if the threshold Z1 between the body tissues L1, L2 reaches the threshold Z1 or a predetermined time t passes after starting to supply energy from the high-frequency energy output portion 104, thereafter the high-frequency energy output portion 104 is made to stop energy output. At this point, collagens are joined on the joined surfaces of the joined portion C of the body tissues L1, L2 due to treatment of the body tissues L1, L2 by high-frequency energy and also the joined surfaces are joined by the substance capable of preventing fluid from infiltrating into the body tissue L_{T}.

The pedal of the foot switch 16 is kept pressed. The body tissue L_{T} also maintains a state of being held by the holding members 52, 54.

A buzz sound or the like is issued from the speaker 110 to tell the end of the treatment (treatment to join body tissues and treatment to prevent fluid from infiltrating to the joined contact surfaces C1, C2) when a predetermined time (for example, a few seconds) passes after the output from the high-frequency energy output portion 104 to the high-frequency electrodes 92, 94 of the first and second holding members 52, 54 is stopped (S16). Then, the physician or the like recognizes the end of treatment based on the sound from the speaker 110 or the display by the display unit 108 and then releases pressing of the pedal by removing a foot from the pedal of the foot switch 16.

The treatment continues from "Start" to "End" shown in FIG. 6 while the pedal of the foot switch 16 is kept pressed, but if the pedal is released at some point between "Start" and "End", the first controller 102 forces the treatment to stop when pressing of the pedal is released. That is, if the supply of high-frequency energy should be stopped in midstream or the supply of adhesive should be stopped in midstream, pressing of the pedal of the foot switch 16 is released by removing a foot from the pedal before a sound such as a buzzer is emitted from the speaker 110. When pressing of the pedal is released, the first controller 102 forces to stop the output of energy from the high-frequency energy output portion 104 to electrodes 92, 94 if the energy is output from the high-frequency energy output portion 104. When the hose 18a is opened, the second controller 132 forces to stop supply of a fluid by causing the flow rate adjustment mechanism 134 to operate to close the hose 18a.

The physician recognizes the buzz sound from the speaker 110 and then operates the treatment portion opening/closing knob 32 to release the body tissue L_{T}. At this point, the contact surfaces C1, C2 of the body tissues are joined to form the joined portion C.

According to the present embodiment, as described above, the following effect is achieved.

Close contact of the contact surfaces C1, C2 of the body tissues L1, L2 can be made more reliable by treating the body tissues L1, L2 for conjugation while measuring the impedance Z of the body tissues L1, L2. Also by disposing the conjugation assistance member 262 between the body tissues L1, L2 to join the body tissues L1, L2, the contact surfaces C1, C2 can be joined by, in addition to a bonding force of the body tissues L1, L2 obtained when high-frequency energy is used, a substance like an adhesive and thus, a large bonding force can be obtained. Because the substance like an adhesive is a substance capable of preventing fluid from invading into the body tissue L_{T}, fluid can be prevented from invading to contact surfaces of the body tissues L1, L2 and thus a large bonding force can be maintained for a long time.

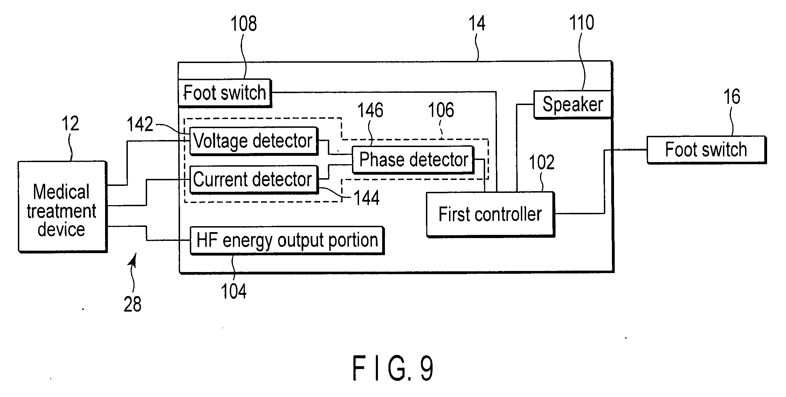

The above embodiment is described as an example of using the impedance Z (see FIG. 5) as living body information detected by the detector 106, but it is also preferable to use the amount of change of the phase (phase difference Δθ) (see FIG. 8) as living body information. A case when the phase difference Δθ is used will be described below as a first modification of the first embodiment with reference to FIGS. 8 and 9.

As shown in FIG. 9, the detector 106 includes a voltage detector 142, a current detector 144, and a phase detector 146. The phase detector 146 is connected to the first controller 102. The voltage detector 142 and the current detector 144 are connected to the energy treatment device 12 (high-frequency electrodes 92, 94) and also connected to the phase detector 146. This is not limited to the first embodiment and applies to other embodiments described later.

If the high-frequency energy output portion 104 is caused to generate a high-frequency voltage, a high-frequency current having a predetermined frequency and peak value based on the high-frequency voltage of the high-frequency energy output portion 104 is output to the surgical treatment device 12 via the current detector 144. The voltage detector 142 detects the peak value of the high-frequency voltage through the high-frequency energy output portion 104 and outputs the detected peak value to the phase detector 146 as output voltage value information. The current detector 144 detects the peak value of the high-frequency current generated based on the high-frequency voltage through the high-frequency energy output portion 104 and outputs the detected peak value to the phase detector 146 as output current value information.

After detecting the phase of the high-frequency voltage output through the high-frequency energy output portion 104 based on output voltage value information output from the voltage detector 142, the phase detector 146 outputs the detected phase to the first controller 102 as output voltage phase information along with output voltage value information. Also after detecting the phase of the high-frequency current through the high-frequency energy output portion 104 based on output current value information output from the current detector 144, the phase detector 146 outputs the detected phase to the first controller 102 as output current phase information along with output current value information.

Based on output voltage value information, output voltage phase information, output current value information, and output current phase information output from the phase detector 146, the first controller 102 calculates the phase difference Δθ of the high-frequency voltage and high-frequency current output through the high-frequency energy output portion 104.

The first controller 102 controls the high-frequency energy output portion 104 to change the output state of the high-frequency current and high-frequency voltage to the ON state or OFF state based on an instruction signal output in accordance with an operation of the pedal of the foot switch 16 and the calculated phase difference Δθ.

As shown in FIG. 8, the phase difference Δθ of the high-frequency current or high-frequency voltage output through the high-frequency energy output portion 104 is 0° or substantially 0° in the initial stage of treatment on the body tissue L_{T}. Incidentally, the value of the phase difference Δθ is set to 90° or a value close thereto through the display unit 108.

As the pedal of the foot switch 16 is pressed uninterruptedly and treatment of the body tissues L1, L2 held between the high-frequency electrodes 92, 94 of the first and second holding members 52, 54 proceeds, the body tissues L1, L2 are dehydrated followed by being cauterized or coagulated. If the treatment proceeds in this manner, the phase difference Δθ of the high-frequency current or high-frequency voltage output through the high-frequency energy output portion 104 increases from the state of 0° or substantially 0°, for example, after a suitable time t1.

Then, if treatment of a desired region proceeds by the pedal of the foot switch 16 being further pressed uninterruptedly, the value of the phase difference Δθ calculated by the first controller 102 takes a fixed value near 90° shown in FIG. 8, for example, after the time t1.

In this modification, the first controller 102 is not limited to the above control exercised when detecting that the phase difference Δθ has become a fixed value near 90° and may be, for example, the above control exercised when detecting that the phase difference Δθ has become a fixed predetermined value greater than 45° and equal to or less than 90°.

Energy input into the body tissues L1, L2 may be switched by combining the change of the impedance Z and the change of the phase θ. That is, it is also preferable to appropriately set by the display unit 108 and use the change of the impedance Z and the change of the phase θ such as a value which is the earlier or the later of reaching a threshold.

Next, an example of using the heaters 222, 232, instead of the electrodes 92, 94, will be described as a second modification of the first embodiment by using

FIGS. 10A to 14. That is, it is assumed that the electrodes 92, 94 shown in FIGS. 3A to 4C are replaced by the heaters 222, 232 shown in FIGS. 10A and 10B.

As shown in FIGS. 10A and 10B, a plate-like heater (energy output portion) 222 is disposed on a main body 62 of a first holding member 52. The heater 222 is enclosed with a holding surface 62a of the main body 62. Though not shown, a plate-like heater (energy output portion) 232 is disposed on a main body 72 of a second holding member 54. The heater 232 is enclosed with a holding surface 72a of the main body 72.

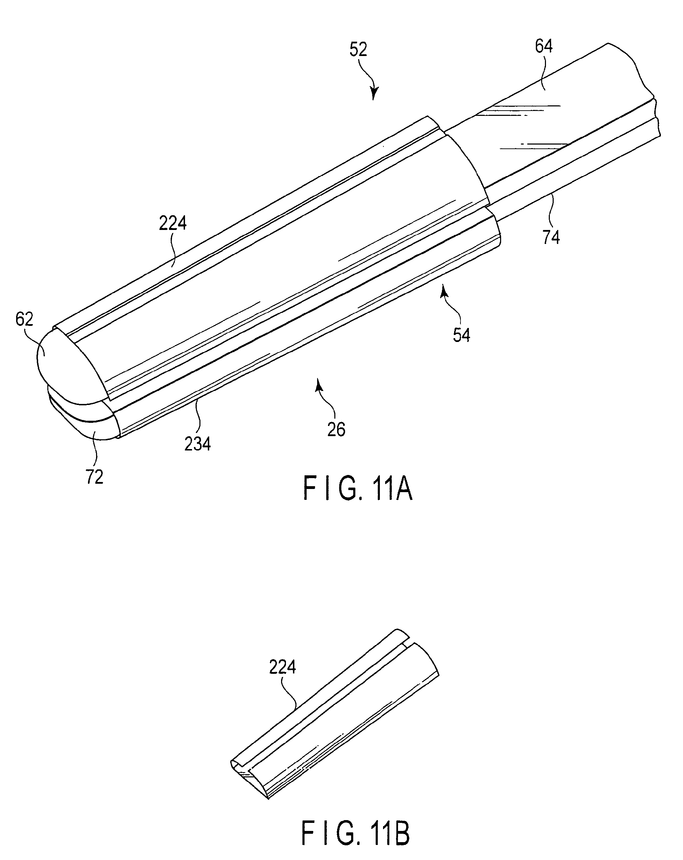

As shown in FIG. 11A, a coating member (sheet-shaped member) 224 (see FIG. 11B) whose transverse section is formed in a C shape in advance is disposed on the outer circumference of the main body 62 of the first holding member 52.

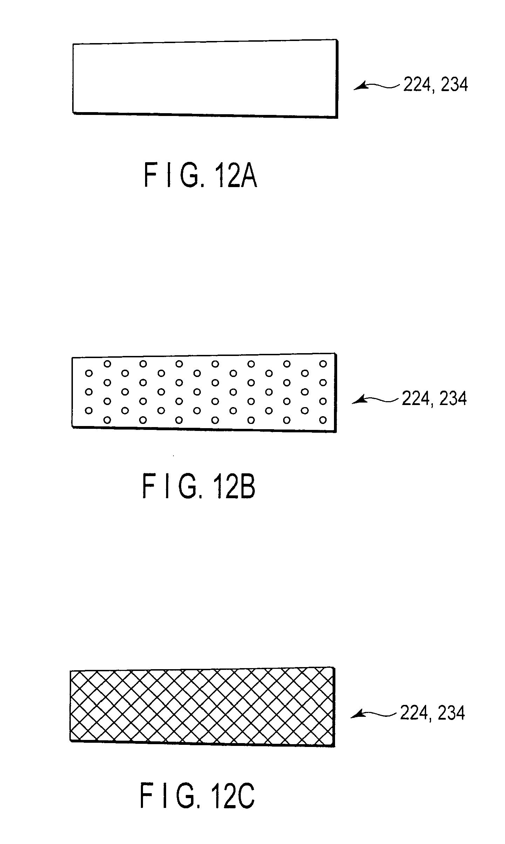

As shown in FIGS. 12A to 12C, a portion of the coating member 224 in contact with the heater 222 has various shapes like non-porous sheet, mesh, and porous shapes. The coating member 224 is formed in the same manner as the above conjugation assistance member 262 and a heated portion thereof is melted when heated to an appropriate temperature and components of the conjugation adjunct spread to the surface of body tissues and invade and are cured while spread on the surface of body tissues and invaded when cooled. When cured, the action of preventing fluid from infiltrating to contact surfaces described later or the like from outside body tissues is achieved.

Incidentally, the coating member 224 is suitably expandable at least in the circumferential direction (width direction perpendicular to the longitudinal direction of the main body 62 of the first holding member 52) before heating (for example, the nonporous sheet-shaped, mesh-shaped, or porous state). Then, when the coating member 224 is disposed on the main body 62 of the first holding member 52, the coating member 224 can be brought into close contact with the holding surface 62a of the main body 62 of the first holding member 52 and an exterior surface of the main body 62 separated from the second holding member 54.

The coating members (a join condition sustainment assistance portion) 224, 234 will be disposed between body tissues L1, L2 and the heaters 222, 232 when the body tissues L1, L2 are held by the main bodies 62, 72 of the first and second holding members 52, 54 and thus the coating members (a conjugation sustainment assistance portion) 224, 234 are pressed toward the heaters 222, 232 by the body tissues L1, L2. Therefore, when the body tissues L1, L2 are held by the first and second holding members 52, 54, the coating members 224, 234 are in contact with the heaters 222, 232.

Ends of the coating member 224 disposed on the first holding member 52 may be opposite to each other in positions of the main body 62 of the first holding member 52 separated from the main body 72 of the second holding member 54 or partially overlapped. The heater 232 and the coating member 234 are also disposed on the second holding member 54. In such a case, the heater 232 and the coating member 234 are suitably disposed on the same manner as in the first holding member 52.

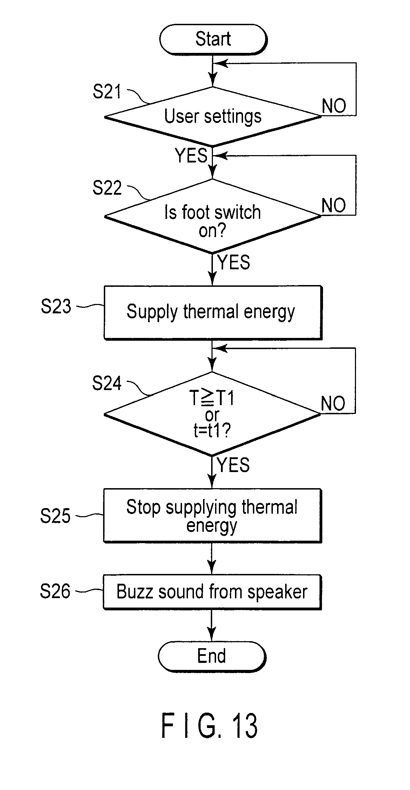

Next, the action of a medical treatment system 10 according to the present embodiment will be described using FIG. 13.

First, a display unit 108 is operated to make various settings. For example, the maximum temperature of the heaters 222, 232, the output time of energy from a high-frequency energy output portion 104 to the heaters 222, 232, a threshold T1 of the end temperature of treatment of body tissues (here, the surface temperature of the body tissues L1, L2) are set (S21).

Then, the body tissues L1, L2 are held by the main bodies 62, 72 of the first and second holding members 52, 54 while the coating members 224, 234 are wound around the main bodies 62, 72 of the first and second holding members 52, 54, respectively. That is, the coating member 224 disposed on the first holding member 52 comes into contact with the surface of the body tissue L1 on the opposite side of the contact surface C1 coming into contact with the body tissue L2. The coating member 234 disposed on the second holding member 54 comes into contact with the surface of the body tissue L2 on the opposite side of the contact surface C2 coming into contact with the body tissue L1.

At this point, the conjugation assistance member 262 in a mesh, porous, or non-porous sheet shape is disposed between the body tissues L1, L2.

If the pedal of the foot switch 16 is pressed in this state (S22), energy is transmitted from the high-frequency energy output portion 104 to the heaters 222, 232 (S23) and the temperature of the heaters 222, 232 gradually rises (electric energy is converted into thermal energy). Then, a portion of the coating members 224, 234 in contact with the heaters 222, 232 melts due to thermal energy of the heaters 222, 232 and a fluid invasion prevention material to the body tissue L_{T} is applied to the exterior surface of the body tissues L1, L2. Also with the rise in temperature of the heaters 222, 232, heat of the heaters 222, 232 is extended to the body tissues L1, L2 to add heat to the body tissues L1, L2.

Then, after an impedance Z is measured, a surface temperature T of the body tissues L1, L2 is measured or a predetermined time t1 passes (S24), the supply of energy from the high-frequency energy output portion 104 to the heaters 222, 232 is stopped (S25). Then, a buzzer sound that tells the end of a sequence of treatment is emitted from a speaker 110 (S26).

The substance that prevents wet from penetrating the body tissue L_{T} is gradually hardened by, for example, being cooled due to stop of supply of energy. Then, the substance that prevents fluid from penetrating the body tissue L_{T} is sustained in a state in which the joined body tissue L_{T} is coated with the substance.

Incidentally, a portion of the coating members 224, 234 that is not in contact with the heaters 222, 232 preferably sustains the state of being disposed on the main bodies 62, 72 of the first and second holding members 52, 54. That is, the side of the coating member 224 disposed on the first holding member 52 separated from the holding surface 62a of the main body 62 with respect to the second holding member 54 sustains the state being disposed on the outer circumferential surface of the main body 62. Also, the side of the coating member 234 disposed on the second holding member 54 separated from the holding surface 72a of the main body 72 with respect to the first holding member 52 sustains the state being disposed on the outer circumferential surface of the main body 72.

If the porous coating members 224, 234 shown in FIG. 12B or the mesh-shaped coating members 224, 234 shown in FIG. 12C are used, instead of the heaters 222, 232, high-frequency electrodes 92, 94 can be used for treatment. If the porous coating members 224, 234 shown in FIG. 12B are used, a portion of the high-frequency electrodes 92, 94 comes into contact with the body tissues L1, L2. If the mesh-shaped coating members 224, 234 shown in FIG. 12C are used, a portion of the high-frequency electrodes 92, 94 comes into contact with the body tissues L1, L2. Therefore, when these porous or mesh-shaped coating members 224, 234 are used, any of the high-frequency electrodes 92, 94 or the heaters 222, 232 can be used.

On the other hand, the nonporous sheet-shaped coating members 224, 234 shown in FIG. 12A are used, the high-frequency electrodes 92, 94 do not come into contact with the body tissues L1, L2 and thus, in this case, it is preferable to use the heaters 222, 232. If the high-frequency electrodes 92, 94 can directly be brought into contact with the body tissues L1, L2 by providing holes in a portion of the nonporous sheet-shaped coating members 224, 234, as will be described below, treatment by high-frequency energy also becomes possible. Also, if the coating members 224, 234 are formed as an energizing member, as will be described below, treatment by high-frequency energy also becomes possible.

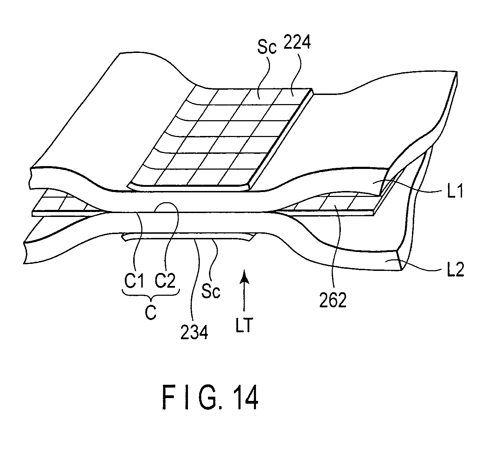

FIG. 14 shows a schematic diagram in which the conjugation assistance member 262 is disposed on the inner side of the body tissues L1, L2 and the outer side thereof is coated with the coating members 224, 234. If the body tissues L1, L2 are treated by using thermal energy or high-frequency energy in such a state, the outer side of the joined body tissue L_{T} is coated with the substance capable of preventing fluid from infiltrating into the body tissue L_{T}.

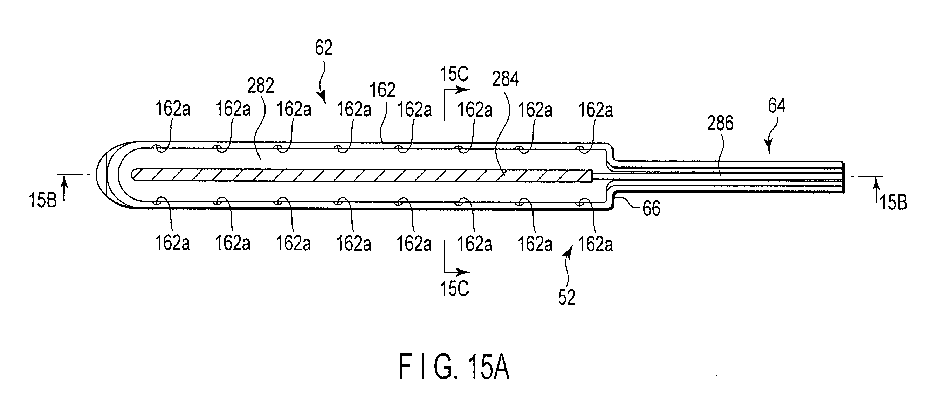

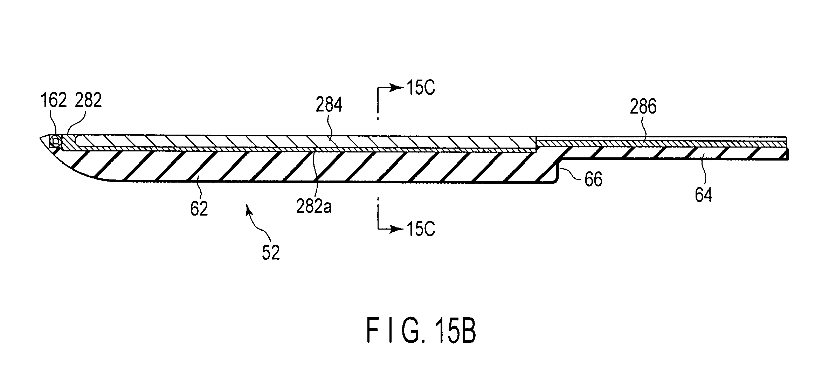

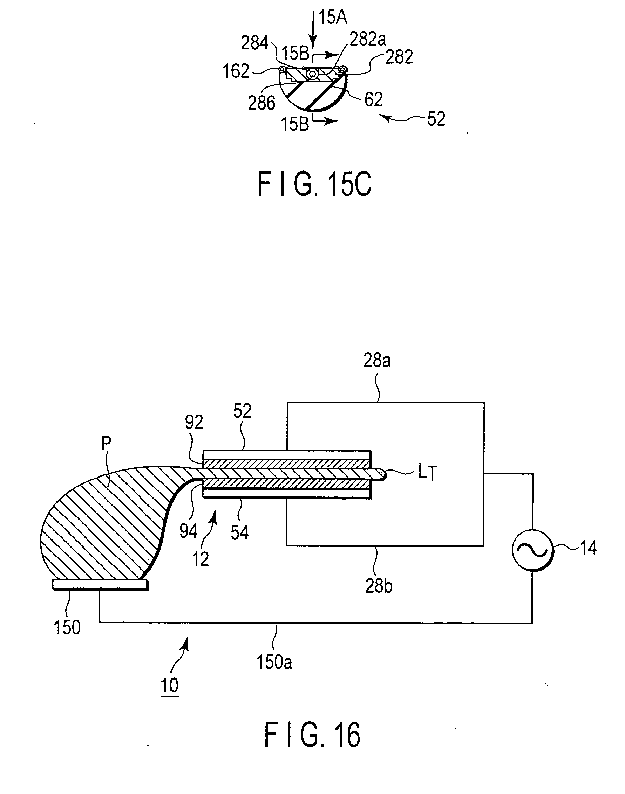

Next, a third modification of the first embodiment will be described using FIGS. 15A to 15C.

While a case when high-frequency energy by the electrodes 92, 94 or thermal energy of heating by the heaters 222, 232 is used for treatment is described in the above embodiment, in the present embodiment, the first holding member 52 of a case when thermal energy by laser light is used for treatment will be described.

As shown in FIGS. 15A to 15C, the first holding member 52 includes a heat exchanger plate (energy output portion) 282, instead of a high-frequency electrode 92, disposed therein. The heat exchanger plate 282 has a concave 282a formed therein. A diffuser 284 as an output member or an energy output portion is disposed in the concave 282a of the heat exchanger plate 282. A fiber (energy output portion) 286 is inserted into the diffuser 284. Thus, if laser light is incident to the fiber 286, the laser light is diffused to the outside from the diffuser 284. Energy of the laser light is converted into thermal energy by the heat exchanger plate 282 being irradiated therewith. Thus, the heat exchanger plate 282 can be used like the heaters 232, 242, as described in the second modification.

A fluid duct 162 shown in FIGS. 15A to 15C has an opening 162a and thus, a substance that prevents fluid from penetrating a body tissue L_{T} can be applied to the outer circumferential surface of the body tissue L_{T}, as described below in the second embodiment.

Instead of the fluid duct 162, an edge (holding surface) 62a of a main body 62 of the first holding member 52 may be formed to carry out treatment using a coating member 224 (see FIGS. 11A and 11B) described in the second modification. That is, treatment can be carried out in the same manner as in the above embodiments when laser light as energy is used.

By using the heat exchanger plate 282 as, for example, the high-frequency electrode 92, various kinds of treatment such as suitable treatment combining thermal energy and high-frequency energy, treatment using only thermal energy, and treatment using only high-frequency energy can be carried out.

A case when the bipolar type energy treatment device 12 is used is described in the first embodiment, but a monopolar type treatment device (see FIG. 16) may also be used in the fourth modification of the first embodiment.

In such a case, as shown in FIG. 16, a return electrode plate 150 is mounted on a patient P to be treated. The return electrode plate 150 is connected to the energy source 14 via an electrical connection line 150a. Further, the high-frequency electrode 92 disposed on the first holding member 52 and the high-frequency electrode 94 disposed on the second holding member 54 are in a state of the same electric potential in which the electrical connection lines 28a, 28b are electrically connected. In this case, each area of the body tissues L1, L2 in contact with the high-frequency electrodes 92, 94 is sufficiently smaller than the area where the return electrode plate 150 is in contact with the living body and so a current density is increased, but the current density in the return electrode plate 150 depresses. Thus, while the body tissues L1, L2 held by the first and second holding members 52, 54 are heated by Joule heat, heating of body tissues in contact with the return electrode plate 150 is so small to be ignorable. Therefore, among the body tissues L1, L2, grasped by the first and second holding members 52, 54, only a portion thereof in contact with the high-frequency electrodes 92, 94 at the same potential is heated and denatured.

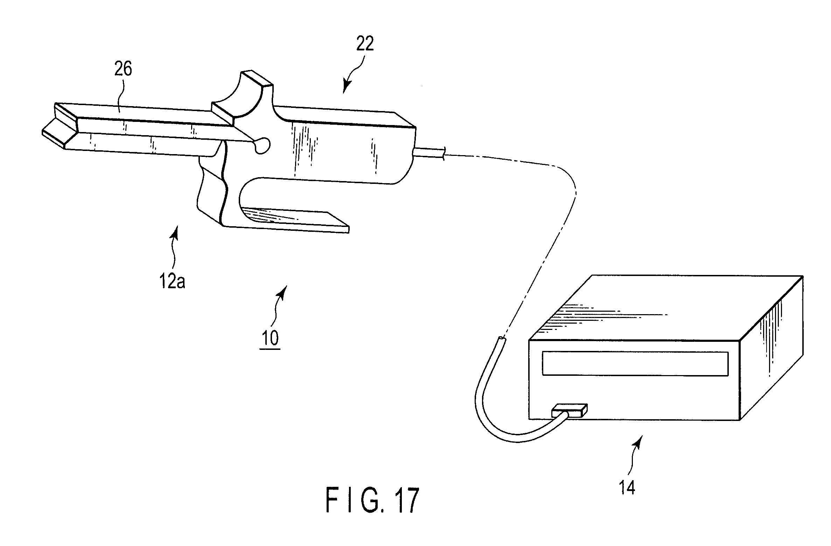

The present embodiment has been described by taking the linear-type energy treatment device 12 (see FIG. 1) to treat the body tissues L1, L2 in the abdominal cavity (in the body) through the abdominal wall as an example, but as shown, for example, in FIG. 17, an open linear-type energy treatment device (medical treatment device) 12a for treatment by taking tissues to be treated out of the body through the abdominal wall may also be used. The energy treatment device 12a includes the handle 22 and the treatment portion (holding portion) 26. That is, in contrast to the energy treatment device 12 (see FIG. 1) for treatment through the abdominal wall, the shaft 24 is removed. On the other hand, a member having the same action as the shaft 24 is disposed inside the handle 22. Thus, the energy treatment device 12a shown in FIG. 17 can be used in the same manner as the energy treatment device 12 shown in FIG. 1 described above.

In the present embodiment, a case when the body tissues L1, L2 are treated by using high-frequency energy has been described, but energy of, for example, a microwave may also be used. In such a case, the high-frequency electrodes 92, 94 can be used as microwave electrodes.

### [Second embodiment]

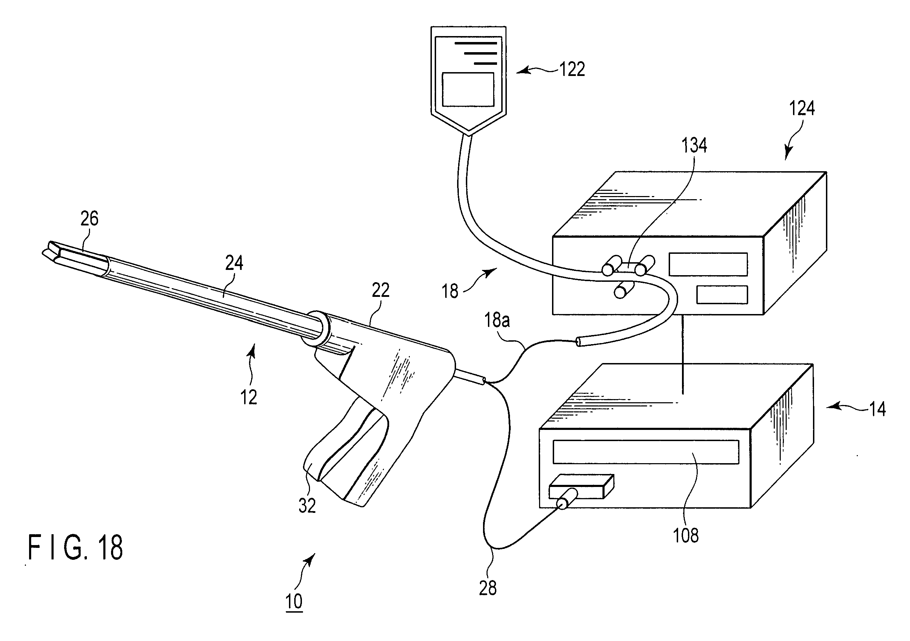

Next, the second embodiment will be described using FIGS. 18 to 21. The present embodiment is a modification of the first embodiment and the same reference numerals are attached to the same members as those used in the first embodiment or members achieving the same action as the action of those in the first embodiment and a description of such members is omitted.

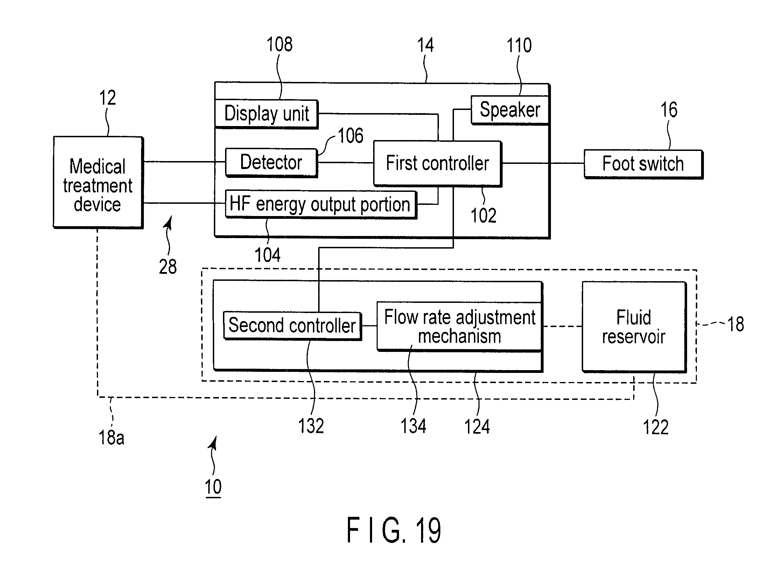

As shown in FIG. 18, in the present embodiment, a medical treatment system 10 includes the energy treatment device 12, an energy source (control section) 14, a foot switch (or a hand switch) 16 (see FIG. 19), and a fluid source 18.

A series of operations such as ON/OFF of the supply of energy (high-frequency energy in the present embodiment) from the energy source 14 to the surgical treatment device 12 and further, whether to make a fluid (conjugation adjunct) flow described later can be switched by the pedal of the foot switch 16 being operated (pressed/released) by an operator. While the pedal is pressed, high-frequency energy is output based on an appropriately set state (state in which the output quantity of energy, timing of energy output and the like are controlled). When pedal pressing is released, the output of high-frequency energy is forced to stop. In addition, a fluid of a predetermined flow rate is made to flow while the pedal is pressed and the flow of the fluid stops when pedal pressing is released.

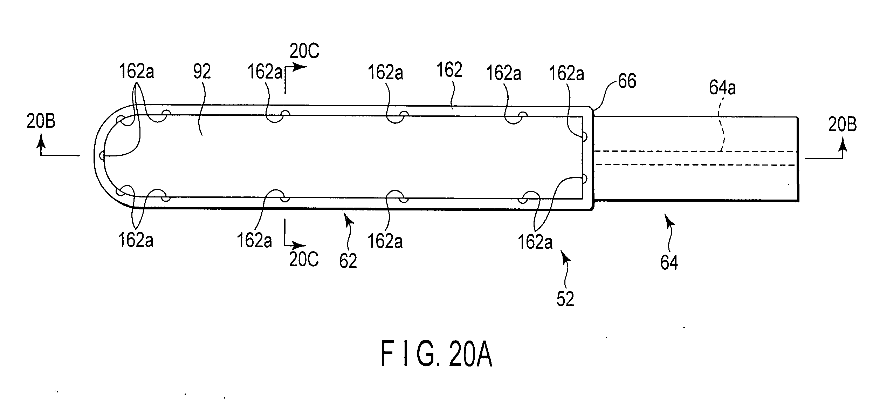

A fluid conduit 162 having insulating properties is disposed on a main body 62 of a first holding member 52 shown in FIGS. 20A to 20C.

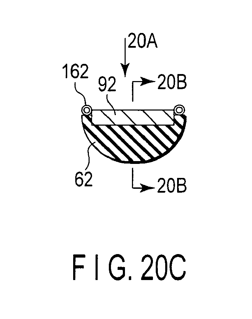

The fluid conduit 162 is disposed on a ring shape in a position close to the surface of the high-frequency electrode 92 along edges of the outer circumference of the main body 62. As shown in FIG. 20C, the transverse section of the fluid conduit 162 is formed, for example, in a circular shape or rectangular shape. The fluid conduit 162 preferably has an appropriate elasticity so as to be in close contact with an exterior surface of the body tissue L1 when the body tissues L1, L2 are held by the first and second holding members 52, 54. The fluid conduit 162 is connected to the duct 64a of the base 64 of the first holding member 52. Incidentally, the high-frequency electrode 92 is disposed inside the fluid conduit 162.

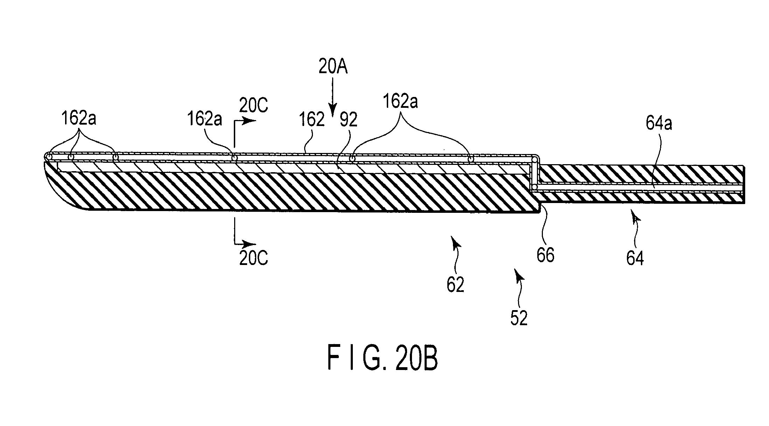

The fluid conduit 162 includes a plurality of openings (a join condition sustainment assistance portion) 162a at suitable intervals. As shown in FIGS. 20B and 20C, these openings 162a are directed toward the surface of the high-frequency electrode 92 and also directed toward the center axis of the high-frequency electrode 92. Thus, a fluid discharged from the openings 162a of the fluid conduit 162 can be passed along the surface of the high-frequency electrode 92 toward the center axis of the high-frequency electrode 92.

Because, as shown in FIG. 20A, the openings 162a of the fluid conduit 162 are positioned close to the surface of the high-frequency electrode 92, a portion of the fluid conduit 162 is projected from the surface of the high-frequency electrode 92. Thus, when the body tissues L1, L2 are treated using the high-frequency electrode 92, the fluid conduit 162 serves as a barrier portion that prevents a fluid such as a steam from being leaked to the outside, the fluid such as a steam being generated from the body tissues L1, L2 when the body tissues L1, L2 are treated using the high-frequency electrode 92.

Though not shown, a fluid conduit 164 having openings (a conjugation sustainment assistance portion) 164a is also disposed at edges of a main body 72 of the second holding member 54 symmetrically with respect to the first holding member 52. Thus, the fluid conduit 164 serves as a barrier portion that prevents a fluid such as a steam from being leaked to the outside, the fluid such as a steam being generated from the body tissues L1, L2 when the body tissues L1, L2 are treated using the high-frequency electrode 94. The fluid conduit 164 is connected to the duct 74a of the base 74 of the second holding member 54.

The fluid source 18 includes a fluid reservoir 122 and a flow rate adjuster 124. The flow rate adjuster 124 includes a second controller (flow rate control unit) 132 and a flow rate adjustment mechanism 134.

The fluid reservoir 122 shown in FIG. 18 is formed from, for example, a transparent bag to store a fluid. The proximal end of the hose 18a is removably connected to the fluid reservoir 122. The second controller 132 of the flow rate adjuster 124 is connected to the first controller 102 of the energy source 14. Therefore, the second controller 132 works by being linked to the energy source 14. The flow rate adjustment mechanism 134 is formed from, for example, a pinch cock so as to adjust the flow rate of a fluid flowing into the energy treatment device 12 through the hose 18a. That is, the second controller 132 controls the flow rate of a fluid such as a liquid supplied from the fluid reservoir 122 to the first and second holding members 52, 54 via the hose 18a by operating the flow rate adjustment mechanism 134.

The fluid reservoir 122 can store a substance (conjugation adjunct) like, for example, an adhesive capable of preventing fluid from invading into the body tissue L_{T} when applied to an exterior surface Sc of the body tissue L_{T} treated by high-frequency energy. The substance capable of preventing fluid from invading into the body tissue L_{T} has been described in the first embodiment and thus, a description thereof is simplified. That is, while an example of using the conjugation assistance member 262 in a mesh or porous shape is described in the first embodiment, the present embodiment is an example in which, in addition to the conjugation assistance member 262 disposed between the body tissues L1, L2, a fluid such as an adhesive having a similar function is applied to the surface of the body tissues L1, L2 and cured.

The substance to be stored in the fluid reservoir 122 may be, in addition to liquids, for example, gel substances. That is, the substance stored in the fluid reservoir 122 may be any fluid that can be passed through the hose 18a. Further, for example, a liquid or gel substance of adhesive stored in the fluid reservoir 122 may contain an antibiotic, growth promoter and the like.

If a liquid substance is stored in the fluid reservoir 122, the liquid substance can be led to the ducts 64a, 74a of the bases 64, 74 and the channels 62b, 72b of the main bodies 62, 72 of the first and second holding members 52, 54 of the energy treatment device 12 through the hose 18a connected to the fluid reservoir 122. If a gel substance is stored in the fluid reservoir 122, the gel substance can be led to the duct 64a of the base 64 and the channel 62b of the main body 62 of the first holding member 52 of the energy treatment device 12 through the hose 18a connected to the fluid reservoir 122 by applying, for example, pneumatic pressure or the like to the fluid reservoir 122.

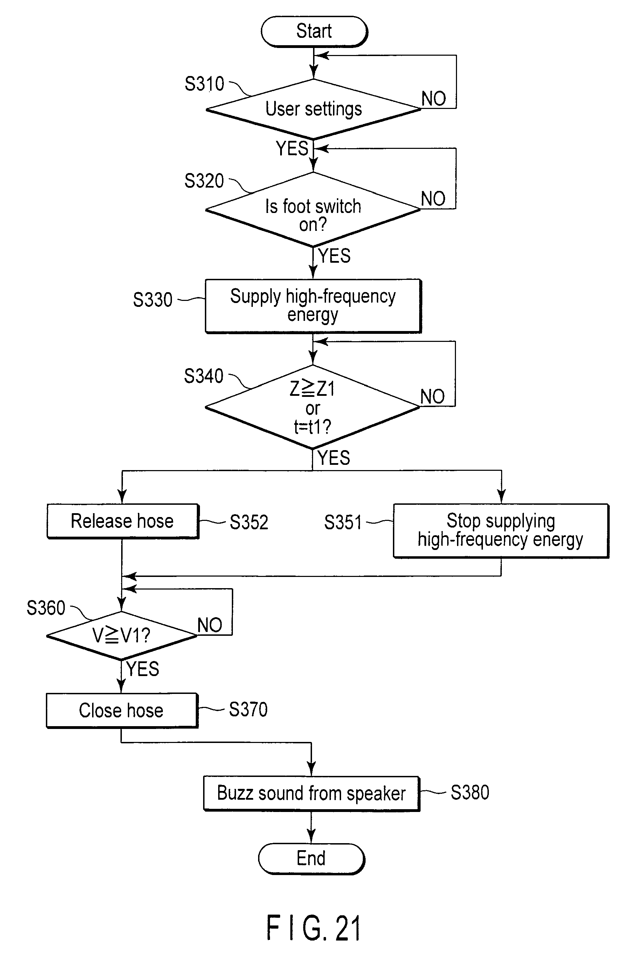

Next, the action of a medical treatment system 10 according to the present embodiment will be described using FIG. 21.

A fluid with which the outer circumference of the body tissue L_{T} obtained by joining the two body tissues L1, L2 is coated after the body tissues L1, L2 being joined by treatment with high-frequency energy is stored in the fluid reservoir 122 of the fluid source 18. It is assumed here that the fluid is an adhesive for the body tissue L_{T}. Particularly, the adhesive suitably has a fast-drying capability that dries when exposed to, for example, the air. The hose 18a connected to the fluid reservoir 122 is closed by the flow rate adjustment mechanism 134 so that no adhesive normally flows from the fluid reservoir 122 toward the energy treatment device 12.

The operator operates the display unit 108 of the energy source 14 in advance to set output conditions for the medical treatment system 10 (step S310). The operator checks the output (set power Pset [W]) from the high-frequency energy output portion 104, the threshold Z1 [Q] of the impedance Z by the detector 106, a maximum energy supply time t1 [sec] and the like through the display unit 108. If the output from the high-frequency energy output portion 104 or the threshold Z1 of the impedance Z by the detector 106 should be set to a different value, the operator sets the value as desired and checks the value through the display unit 108. The operator also sets a flow rate V1 to be passed from the fluid reservoir 122 to the energy treatment device 12 through the hose 18a. Further, the operator sets a longest time t-max in which the hose 18a is opened. That is, even if the flow rate V1 is not reached after the hose 18a is opened, the hose 18a is automatically closed after the time t-max passes.

Then, as described in the first embodiment, the body tissue L_{T} having the joined portion C is formed by holding the body tissues L1, L2 to be joined between the first holding member 52 and the second holding member 54 to join the two body tissues L1, L2 (S320 to S340).

The supply of energy from the high-frequency energy output portion 104 to the high-frequency electrodes 92, 94 is stopped by the first controller 102 (S351) and at the same time, a signal is conveyed from the first controller 102 to the second controller 132. The second controller 132 causes the flow rate adjustment mechanism 134 to operate to open the hose 18a (S352). Thus, an adhesive is supplied from the fluid reservoir 122 to the energy treatment device 12 through the hose 18a. That is, the adhesive is supplied from the fluid reservoir 122 to the ducts 64a, 74a of the bases 64, 74 and the channels 62b, 72b of the main bodies 62, 72 of the first and second holding members 52, 54 by the hose 18a through inner portions of the handle 22 and the shaft 24. Thus, the adhesive is oozed out from the openings 92a, 94a of the high-frequency electrodes 92, 94 formed along the channels 62b, 72b of the main bodies 62, 72.

The adhesive oozed out from the openings 92a, 94a of the high-frequency electrodes 92, 94 is spread and applied to coat the outer circumferential surface of joined body tissues. That is, the adhesive is applied to the entire surface through which the high-frequency electrodes 92, 94 and body tissues are in contact. Then, the adhesive is gradually hardened with the passage of time if, for example, exposed to the air. The adhesive preferably has a quick-drying capability and has waterproof when hardened. Thus, the exterior surface Sc of the body tissue L_{T} joined with hardening of the adhesive is coated. Therefore, a liquid can be prevented from infiltrating from the exterior surface Sc of the joined body tissue L_{T} into the joined portion C (between the contact surfaces C1, C2).

Adhesives have naturally different properties depending on the type of adhesive and the reason why the adhesive in the present embodiment is applied after the body tissues L1, L2 are joined is that an adhesive for body tissues can display an effective adhesive action when applied in as dry a state of the body tissues L1, L2 as possible. That is, if an adhesive is applied in a state in which a sufficient amount of fluid is not removed, it becomes more difficult to remove fluid from the body tissues L1, L2 even if energy is provided, but such a state can be prevented by applying the adhesive after the body tissues L1, L2 are joined. In addition, if an adhesive is applied in a state in which a sufficient amount of fluid is not removed, the adhesive may be mixed with fluid, but such a state can be prevented by applying the adhesive after the body tissues L1, L2 are joined.

When the adhesive of a predetermined flow rate is passed from the fluid reservoir 122 through the hose 18a (S360) or after the hose 18a is opened for a predetermined time, the second controller 132 causes the flow rate adjustment mechanism 134 to operate again to close the hose 18a (S370).

When a predetermined time (for example, a few seconds) passes after the hose 18a is closed, a sound such as a buzzer from the speaker 110 is emitted to tell the completion of treatment (conjugation treatment of body tissues and treatment to prevent fluid from invading into the joined contact surfaces C1, C2) (S380). Then, after making sure that the treatment has completed with the sound from the speaker 110 or the display of the display unit 108, a medical doctor or the like releases the pedal by removing his or her foot from the pedal of the foot switch 16.

Though not shown, the fluid conduit 162 is preferably formed as a double lumen so that one (inner side) is a duct having the openings 162a and the other (outer side) is a duct that passes a gas or liquid as a refrigerant. In this case, a portion of the body tissues L1, L2 in contact with the fluid conduit 162 can be cooled by circulating a refrigerant through the other duct (duct on the outer side). Therefore, heat can be prevented from conducting to the outer side of the holding surfaces 62a, 72a of the first and second holding members 52, 54 through the body tissues L1, L2 so that the body tissues L1, L2 outside the body tissues L1, L2 to be treated can more reliably be prevented from being affected by heat.

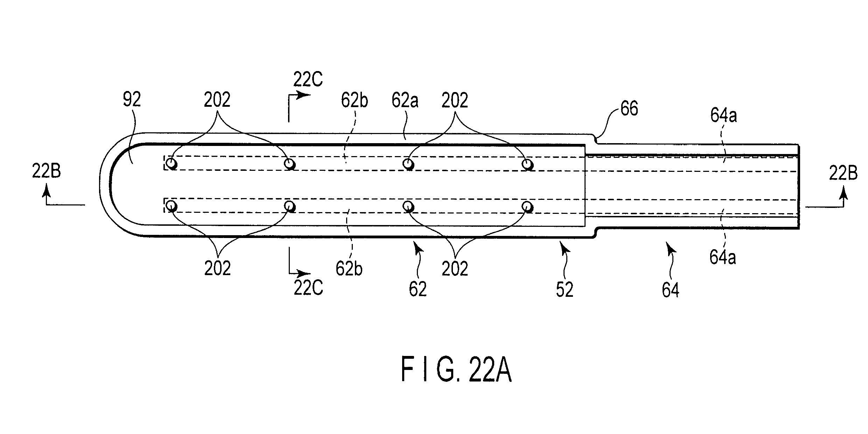

Next, a first modification of the second embodiment will be described using FIGS. 22A to 24A. The present modification is an example of directly introducing an adhesive into between contact surfaces C1, C2 of the body tissues L1, L2 without using the conjugation assistance member 262.

As is shown in FIG. 22A to 22C, the main body 62 of the first holding member 52 has two rows of the flow paths (channels) 62b preferably parallel to each other formed in a concave shape. That is, the channel 62b of the main body 62 is open to the outer side. The tip of the channel 62b is closed.

Two rows of the ducts 64a preferably parallel to each other are formed in the base 64. That is, the duct 64a of the base 64 is closed to the outside excluding both ends. The channel 62b of the main body 62 and the duct 64a of the base 64 are formed successively. The tip of the hose 18a inserted into the shaft 24 and having pliability is connected to the end face of the duct 64a of the base 64.

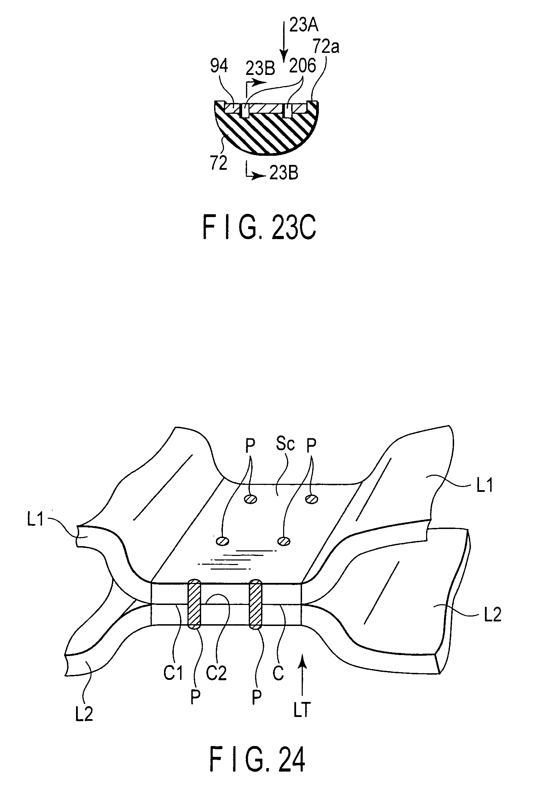

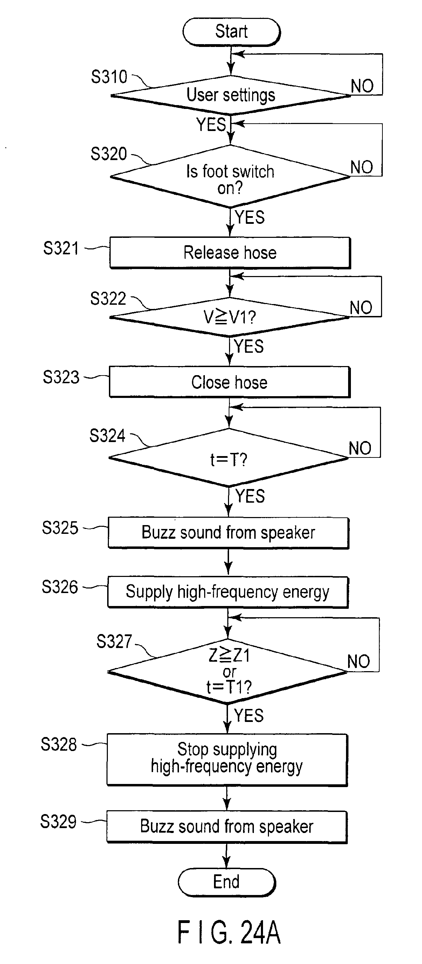

The first high-frequency electrode 92 described above is disposed in the main body 62 of the first holding member 52 like putting a lid. The first high-frequency electrode 92 has a plurality of projections (conjugation maintenance assistance portions) 202 toward the second holding member 54 over the recess 62b of the main body 62 of the first holding member 52. The projection 202 is formed to an appropriate length so as to form a hole P shown in FIG. 24 in the body tissues L1, L2. The projection 202 does not necessarily need to cut through the body tissues L1, L2 and the tip (distal end with respect to the first high-frequency electrode 92) of the projection 202 is suitably positioned closer to the second high-frequency electrode 94 than the contact surfaces C1, C2 of the body tissues L1, L2.

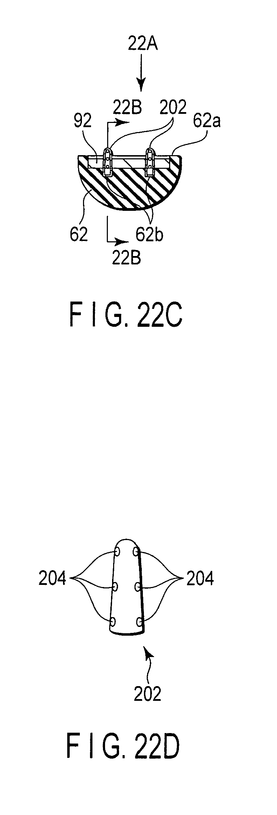

As shown in FIG. 22D, each of the projections 202 has one or a plurality of openings 204 formed therein. The plurality of openings 204 is preferably formed. The projection 202 is communicatively connected to the recess 62b and a fluid (conjugation adjunct) such as an adhesive can be oozed out through the recess 62b.

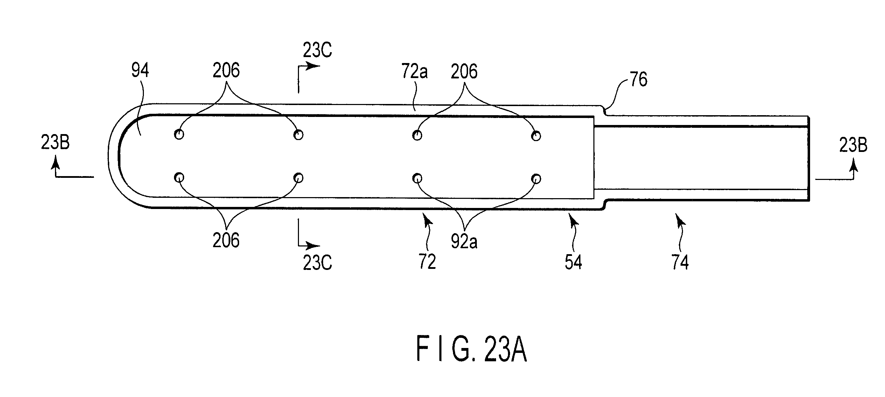

As shown in FIGS. 23A to 23C, a main body 72 of the second holding member 54 and the high-frequency electrode 94 have recesses (a join condition sustainment assistance portion) 206 formed therein. Each of the recesses 206 is formed so as to accommodate the projection 202 disposed on the first holding member 52 and projecting from the high-frequency electrode 92.

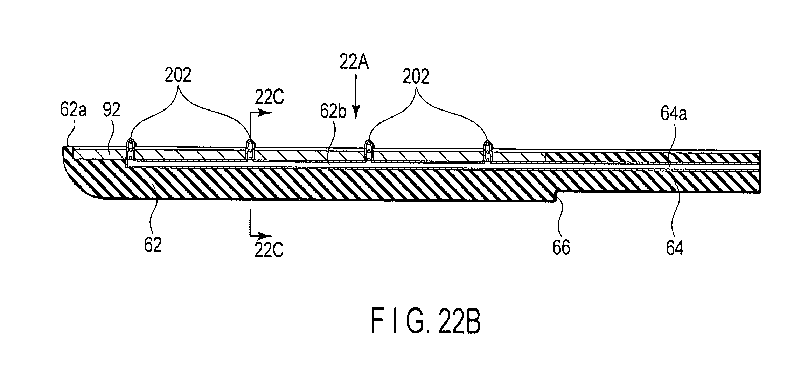

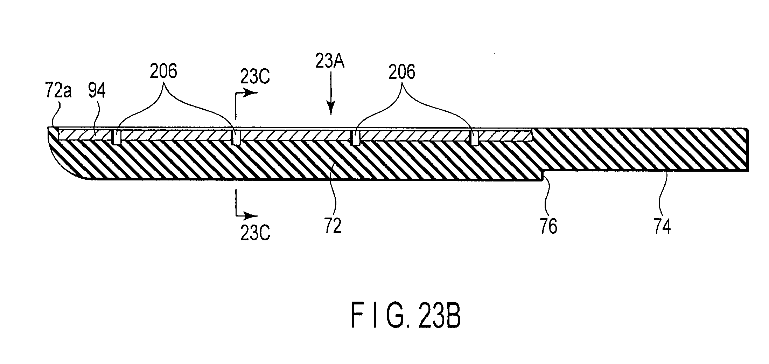

As shown in FIGS. 22B, 22C, 23A and 23B, the surface of the high-frequency electrodes 92, 94 is positioned lower than edges 62a, 72a of the main bodies 62, 72 of the first and second holding members 52, 54. The length of the projection 202 of the first high-frequency electrode 92 is formed to a height that does not come into contact with the recess 206 of the second holding member 54. Thus, the first high-frequency electrode 92 and the second high-frequency electrode 94 are formed so as not to come into contact with each other even if the projection 202 of the first high-frequency electrode 92 is disposed in the recess 206 of the second high-frequency electrode 94.

Next, the medical treatment system 10 according to the present embodiment is caused to operate in the same manner as in the second embodiment to treat the body tissues L1, L2 for conjugation.

Like in the second embodiment, the body tissues L1, L2 to be joined are held. In this case, the projections 202 are disposed on the high-frequency electrode 92 disposed on the first holding member 52 and thus, the projections 202 form the holes P by passing through the body tissues L1, L2 and also are accommodated in the recesses 206 disposed on the second holding member 54 and the high-frequency electrode 94.

In this state, the two body tissues L1, L2 are joined by high-frequency energy output from the high-frequency electrodes 92, 94 disposed on the first and second holding members 52, 54. At this point, the projections 202 provided on the high-frequency electrode 92 disposed on the first holding member 52 sustain a state of passing through the body tissues L1, L2 (state disposed in the hole P).

In this case, the projections 202 are disposed inside the body tissues L1, L2 and power is passed through body tissues between the projections 202 and the second high-frequency electrode 94 and therefore, treatment of the body tissues L1, L2 using high-frequency energy can be carried out efficiently.

After an impedance Z reaches a threshold Z1, a flow rate adjustment mechanism 134 is released to allow an adhesive to flow from a fluid reservoir 122 through a hose 18a. In this case, a duct 64a is provided in a base 64 of the first holding member 52 and the recess 62b is provided in the main body 62 and thus, an adhesive is oozed out from the openings 204 of the projections 202. In this case, the projections 202 are disposed in the holes P by passing through the joined body tissue L_{T} and thus, a portion of the adhesive oozed out from the openings 204 is applied to the joined portion C of the body tissue L_{T}. A portion of the adhesive penetrates directly through the joint surface of the joined portion C. The adhesive has, in addition to the adhesive action, the coating action and thus, fluid can be prevented from infiltrating into the joined portion C and also the joined state can be sustained.

When a sequence of the treatment of the conjugation of the body tissues L1, L2 by high-frequency energy and the application of the adhesive to the joined portion C is completed, a sound such as a buzzer sound is emitted from a speaker 110 to let the medical doctor know completion of the treatment.

According to the present embodiment, as described above, the following effect is achieved.

Because Joule heat can be generated not only in the body tissues L1, L2 between the high-frequency electrodes 92, 94, but also in the body tissues L1, L2 between the projections 202 passing through the body tissues L1, L2 and the high-frequency electrode 94 and thus, it can be made easier for energy to penetrate the body tissues L1, L2 even if the body tissues L1, L2 are thick (if it is difficult for high-frequency energy to penetrate the body tissues L1, L2).

Because a fluid such as an adhesive can directly be supplied into the joined body tissue L_{T} such as the joined portion C of the body tissues L1, L2 to be joined for invasion by the projections 202 provided on the high-frequency electrode 92, the conjugation of the joined portion C can be made more reliable and also the coating action of the adhesive can be extended to the neighborhood of the joined portion C including the joint surface.

In the present embodiment, a case when the holes P are formed in the body tissues L1, L2 by the projections 202 of the first holding member 52 when body tissues are held by the first and second holding members 52, 54 has been described. However, when the body tissues L1, L2 are held by the first and second holding members 52, 54, the holes P do not necessarily need to be formed by the projections 202. That is, when the body tissues L1, L2 are held by the first and second holding members 52, 54, the projections 202 of the first holding member 52 may be provided in such a way that the body tissue L2 is pressed against the recesses 206 of the second holding member 54. Also in this case, with the supply of high-frequency energy to the body tissues L1, L2 between the first and second high-frequency electrodes 92, 94, the holes P will be formed in the body tissues L1, L2, that is, the projections 202 will be disposed in the holes P.

The projections 202 of the high-frequency electrode 92 of the first holding member 52 may be formed as a different body such as a hardening resin material having insulating properties. In this case, the projections 202 are permitted to come into contact with the high-frequency electrode 94 of the second holding member 54.

The discharge of the adhesive from the opening 204 of the projection 202 is not limited to after dehydration (after conjugation) of the body tissues L1, L2 and the adhesive may be applied to between the joined surfaces C1, C2 of the body tissues L1, L2 before dehydration (before conjugation).

A sequence of treatment of a case when the adhesive is applied to between the joined surfaces C1, C2 of the body tissues L1, L2 before dehydration (before conjugation) will briefly be described below using FIG. 24A.

The operator operates the display unit 108 of the energy source 14 in advance to set output conditions of the medical treatment system 10 (step S310). Here, the output (set power Pset [W]) from the high-frequency energy output portion 104, the threshold Z1 [Ω] of the impedance Z of the detector 106, an energy maximum supply time t1 [sec], and further a T1 [sec] till infiltration of the adhesive in the body tissues L1, L2 after the hose 18a is closed are checked via the display unit 108.

Then, the body tissues L1, L2 to be joined are held between the first holding member 52 and the second holding member 54 and the foot switch 16 is changed to ON (S320).

Then, the hose 18a is released (S321) and an adhesive is applied to between the joint surfaces C1, C2 of the body tissues L1, L2 through the opening 204 of the projection 202. When a predetermined discharge of adhesive flows through the hose 18a (S322), the hose 18a is closed (S323). Then, the invasion of the adhesive into the body tissues L1, L2 from the joint surfaces C1, C2 is awaited. That is, when the time T1 passes after the hose 18a is closed (S324), a buzz sound to tell the operator that the body tissues L1, L2 are invaded after the adhesive being applied to the contact surfaces C1, C2 and next, high-frequency energy is supplied to the body tissues L1, L2 is issued from the speaker 110 (S325).

Then, high-frequency energy is supplied to the body tissues L1, L2 and after predetermined treatment is provided, a buzz sound is issued from the speaker 110, which allows the operator to recognize that a sequence of treatment has been completed (S326 to S329).

After the hose 18a is released, the hose 18a may be closed while high-frequency energy being supplied.

Thus, in which period of before conjugation, during conjugation, and after conjugation to discharge an adhesive stored in the fluid reservoir 122 of the fluid source 18 to the body tissues L1, L2 through the hose 18a can appropriately set by the second controller 132.

### [Third embodiment]

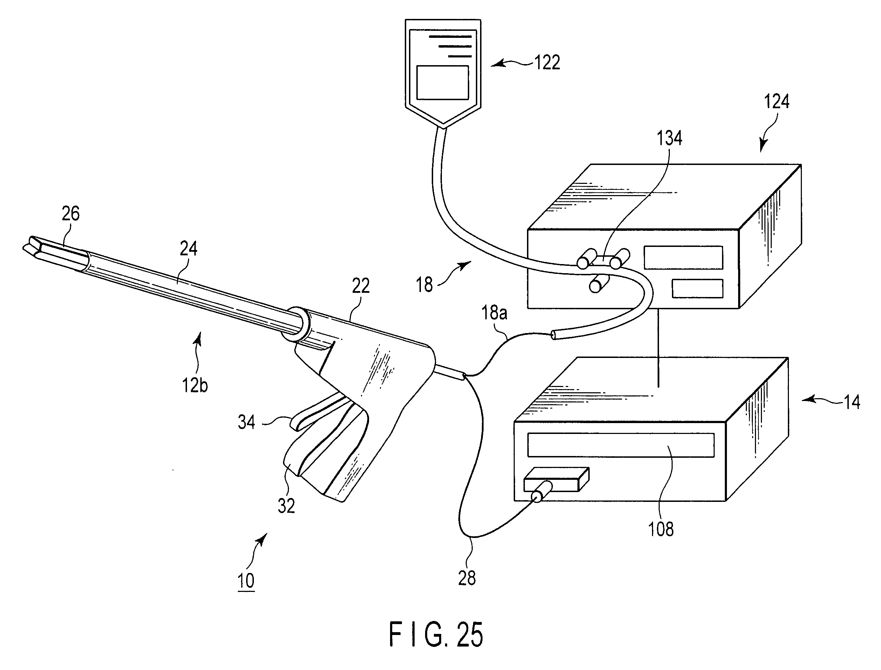

Next, the third embodiment will be described using FIGS. 25 to 30. The present embodiment is a modification of the first and second embodiments and the same reference numerals are attached to the same members as those used in the first and second embodiments or members achieving the same action as the action of those in the first and second embodiments and a description of such members is omitted.

As shown in FIG. 25, a handle 22 of an energy treatment device 12b includes a cutter driving knob 34 to move a cutter (auxiliary treatment device) 180 described later while being installed adjacent to the treatment portion opening/closing knob 32.

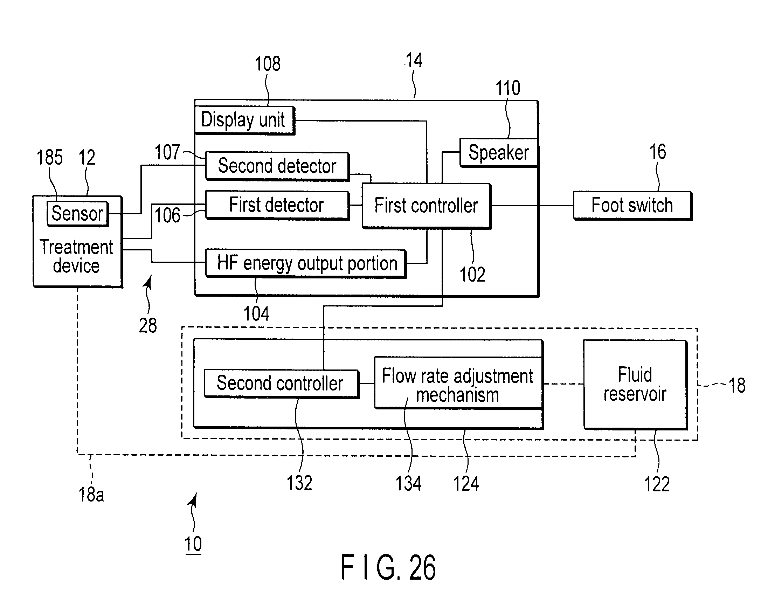

As described in FIG. 26, in addition to a detector (called a first detector here) 106 described in the first embodiment, a second detector 107 is connected to a first controller 102 in an energy source 14. The second detector 107 is connected to a sensor 185 disposed in locking portions 184a, 184b, 184c of a long groove 184 described later of the cutter 180.

The external shapes of main bodies 62, 72 and bases 64, 74 of first and second holding members 52, 54 are formed similarly to the external shapes of the first and second holding members 52, 54 (see FIGS. 20A to 20C) in the second embodiment except that cutter guiding grooves 172, 174 described later are formed.

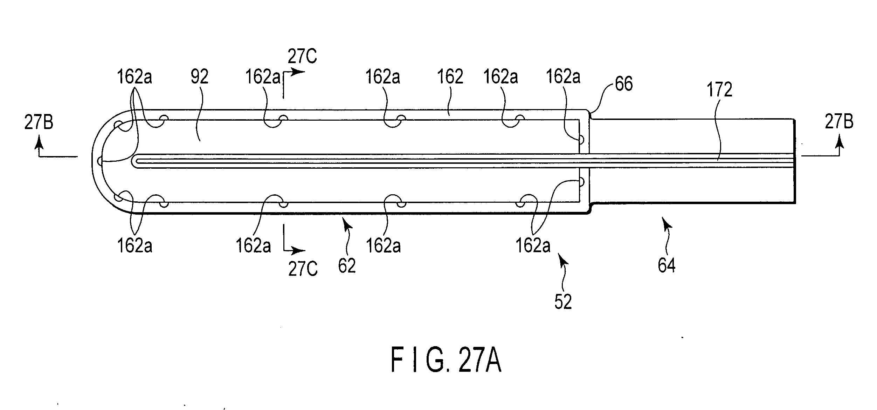

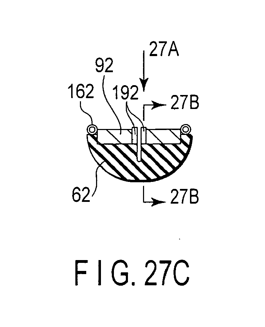

As shown in FIGS. 27A to 28B, the straight cutter guiding groove 172 is formed on the main body 62 and the base 64 of the first holding member 52 closer to the second holding member 54. Similarly, the straight cutter guiding groove 174 is formed on the main body 72 and the base 74 of the second holding member 54 closer to the first holding member 52. The cutter 180 described later is configured to advance to/retreat from these cutter guiding grooves 172, 174.

As shown in FIG. 27A, high-frequency electrodes 92, 94 disposed on the main bodies 62, 72 of the first and second holding members 52, 54 are formed, for example, in a substantial U shape and each have two ends in the proximal end of the main bodies 62, 72 of the first and second holding members 52, 54. That is, each of the high-frequency electrodes 92, 94 is formed continuously. The high-frequency electrodes 92, 94 have cutter guiding grooves (reference numerals 172, 174 are conveniently attached) to guide the cutter 180 formed together with the first and second holding members 52, 54.

The cutter guiding grooves 172, 174 of the first and second holding members 52, 54 are formed in a mutually opposite state along the axial direction of a shaft 24. Then, the cutter 180 can be guided by the two collaborating cutter guiding grooves 172, 174 of the first and second holding members 52, 54.

A driving rod 182 is movably disposed inside a pipe 42 of the shaft 24 along the axis direction thereof. The cutter driving knob 34 is disposed at the proximal end of the driving rod 182. The cutter (auxiliary treatment device) 180 in a thin plate shape is disposed at the tip end of the driving rod 182. Thus, if the cutter driving knob 34 is operated, the cutter 180 moves along the axial direction of the shaft 24 via the driving rod 182.

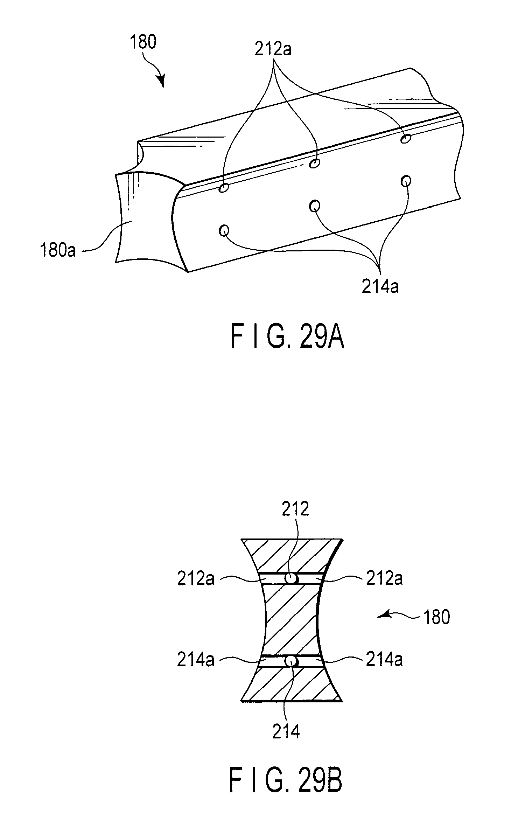

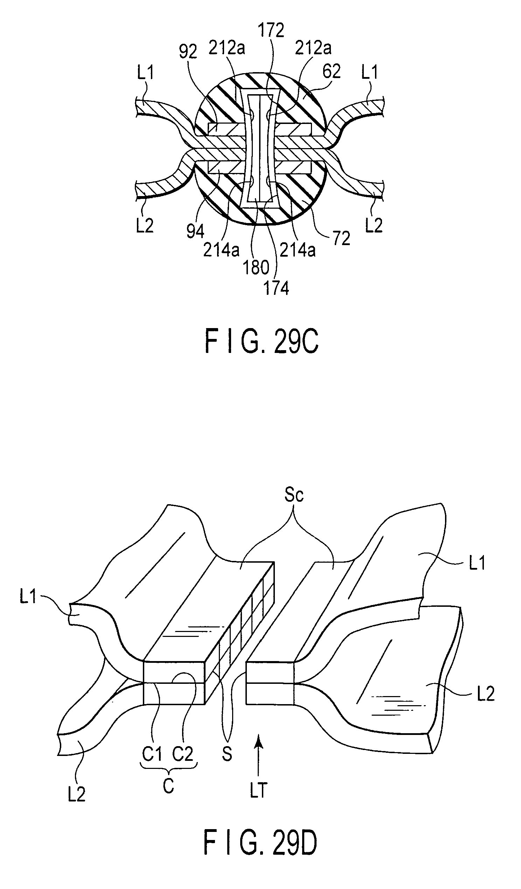

A cutter 180 shown in FIG. 29A has a cutting edge 180a at the tip end thereof. The cutter 180 has ducts 212, 214 formed, for example, shown in the upper and lower parts in FIGS. 29A and 29B, inside along the longitudinal direction of the cutter 180. The ducts 212, 214 formed inside the cutter 180 are connected to a hose 18a through an inner portion of a driving rod 182. As shown in FIGS. 29A and 29B, a plurality of openings (conjugation sustainment assistance portions) 212a, 214a are formed at suitable intervals along the longitudinal direction of the cutter 180 on the side face of the cutter 180. These openings 212a, 214a are communicatively connected to the ducts 212, 214. Thus, a fluid infiltration prevention substance (conjugation adjunct) to a body tissue L_{T} such as an adhesive can be discharged from the openings 212a, 214a through the ducts 212, 214.

The tip end of the driving rod 182 is fixed to the proximal end of the cutter 180. A long groove 184 is formed between the tip end and the proximal end of the cutter 180. In the long groove 184, a movement regulation pin 42a extending in a direction perpendicular to the axial direction of the shaft 24 is fixed to the pipe 42 of the shaft 24. Thus, the long groove 184 of the cutter 180 moves along the movement regulation pin 42a. Therefore, the cutter 180 moves straight. At this point, the cutter 180 is disposed in the cutter guiding grooves (channels, fluid discharge grooves) 172, 174 of the first and second holding members 52, 54.

The locking portions 184a, 184b, 184c to control the movement of the cutter 180 by locking the movement regulation pin 42a are formed, for example, at three locations of one end, the other end, and therebetween. The sensor 185 capable of recognizing the position of the movement regulation pin 42a and also recognizing the direction of movement of the movement regulation pin 42a is disposed in the long groove 184 of the cutter 180. Various kinds of sensors such as a sensor using light and a contact type sensor are used as the sensor 185. Thus, it becomes possible to recognize that the cutting edge 180a of the cutter 180 is contained in the shaft 24 when the movement regulation pin 42a is positioned in the locking portion 184a at the one end (tip end) of the long groove 184 and the cutting edge 180a of the cutter 180 is disposed in the cutter guiding grooves 172, 174 through the tip end of the shaft 24 when the movement regulation pin 42a is positioned at the other end (rear end) 184b. Therefore, the second detector 107 can recognize the position of the cutting edge 180a of the cutter 180 with respect to the shaft 24 and a treatment portion 26 through the sensor 185 and can easily determine whether the cutting edge 180a of the cutter 180 is in a position to cut body tissues.

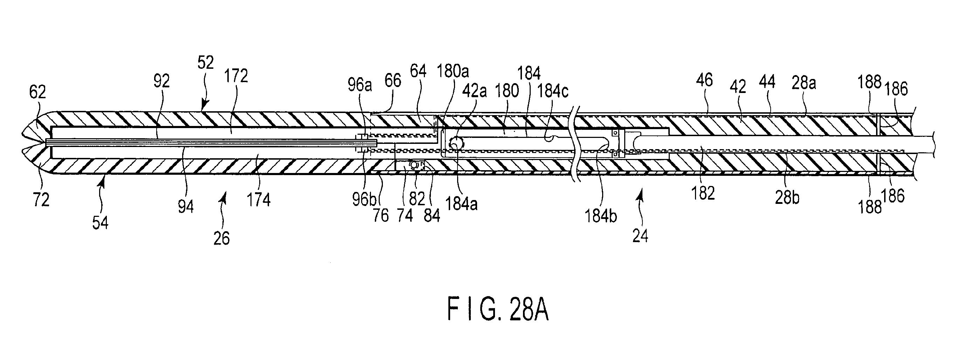

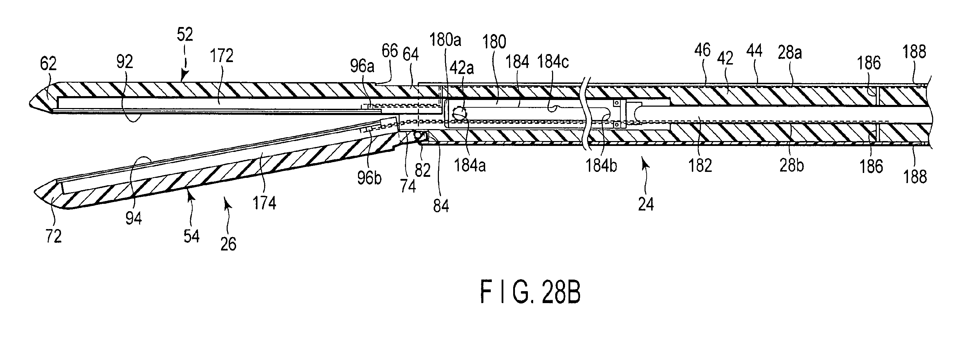

The pipe 42 and a sheath 44 of the shaft 24 of the energy treatment device 12 shown in FIGS. 28A and 28B include fluid discharge ports 186, 188 through which a fluid such as a steam (gas) or liquid (tissue fluid) described later is discharged formed respectively. These fluid discharge ports 186, 188 are formed on the rear end side of the shaft 24.

Though not shown, a connection mouthpiece is suitably provided on the outer circumferential surface of the fluid discharge port 188 of the sheath 44. At this point, the fluid described later is discharged through the cutter guiding grooves 172, 174, the fluid discharge port 186 of the pipe 42 of the shaft 24, the fluid discharge port 188 of the sheath 44 of the shaft 24, and the connection mouthpiece. In this case, a fluid such as a steam and liquid released from body tissues L1, L2 can easily be discharged from the fluid discharge ports 186, 188 by sucking from inside the connection mouthpiece.

The fluid discharge ports 186, 188 are suitably provided in the shaft 24, but may also be suitably provided in the handle 22.

As shown in FIGS. 27A to 27C, first fluid conduits 162, 164 (described simply as the fluid conduits 162, 164 in the second embodiment) are disposed on the main bodies 62, 72 of the first and second holding members 52, 54, which has been described in the second embodiment and a description thereof is omitted.

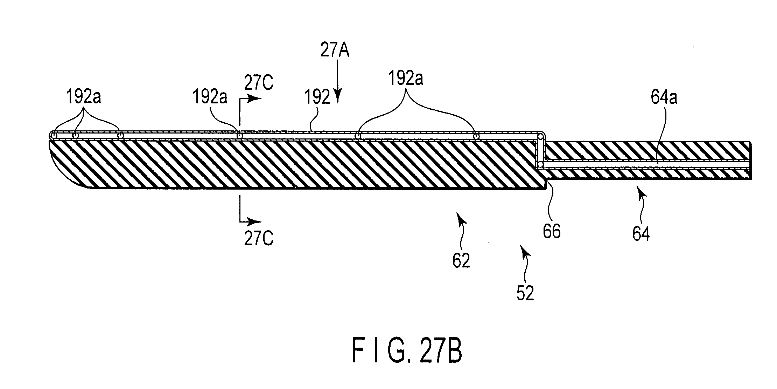

As shown in FIG. 27B, second fluid conduits 192, 194 having insulating properties are disposed at edges of the cutter guiding grooves 172, 174. The second fluid conduit 192 is connected to, for example, a duct 64a of the base 64 of the first holding member 52. Similarly, the other second fluid conduit 194 is connected to, for example, a duct 74a of the base 74 of the second holding member 54.

The second fluid conduits 192, 194 each have a plurality of openings (join condition sustainment assistance portions) 192a, 194a formed at suitable intervals. The openings 192a, 194a of the fluid conduits 192, 194 are oriented toward the same second fluid conduits 192, 194 opposite to each other across the cutter 180.

Incidentally, the second fluid conduits 192, 194 may each be a pair or respective individual conduit bents in a U shape.

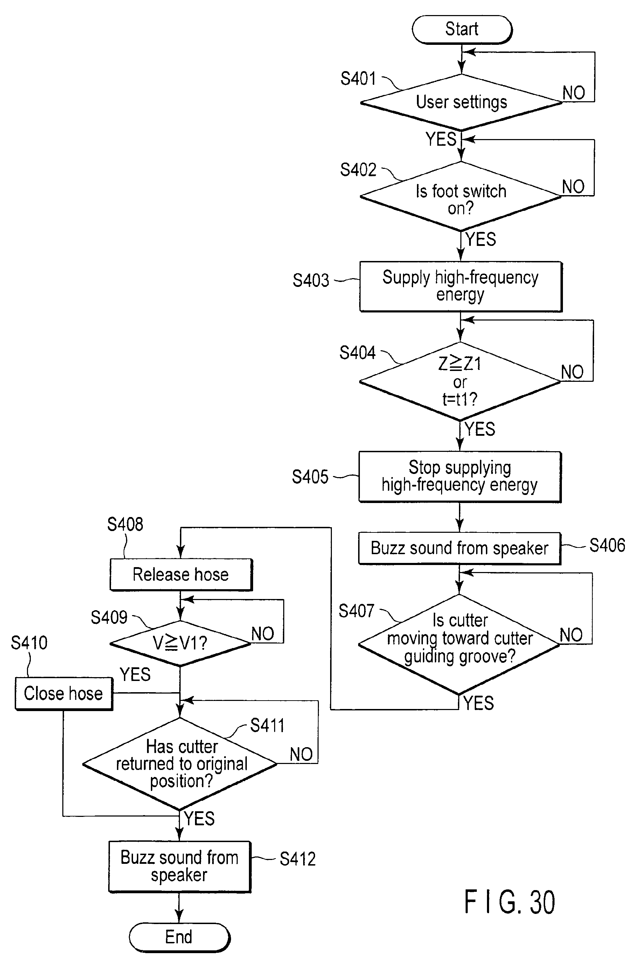

Next, the action of a medical treatment system 10 according to the present embodiment will be described using FIG. 30.

As described in the second embodiment, the contact surfaces C1, C2 of the body tissues L1, L2 are joined by high-frequency energy provided by the high-frequency electrodes 92, 94 (S401 to S406).

Then, the cutter 180 is caused to operate to cut the joined body tissue L_{T} (S407). The hose 18a is opened in conjunction with the operation of the cutter 180 (S408). Thus, while the joined body tissue L_{T} is cut, an adhesive is made to ooze out from the opening 212a of the cutter 180 to apply the adhesive to a cut surface S. That is, as cutting of the body tissue L_{T} proceeds, an adhesive oozed out from the opening 212a of the cutter 180 is applied.

Because, as shown in FIG. 29B, the openings 212a are formed in an upper portion and a lower portion of the cutter 180 and thus, if it is assumed that the body tissues L1, L2 have the same thickness, the adhesive is applied to a position deviating from joint surfaces of the joined portion C. The applied adhesive flows in an appropriate direction depending on the orientation of the first and second holding members 52, 54 and thus, the adhesive is applied to the whole cut surface S by the cutter 180.

The adhesive also flows to the surface in contact with the high-frequency electrodes 92, 94 of the body tissue L_{T} to be applied there. Thus, the adhesive is applied to the whole exterior surface of the body tissue L_{T}.

When a predetermined discharge of adhesive flows through the hose 18a (S409), the hose 18a is closed (S410) and also the cutter 180 is caused to return to the original position thereof. Then, if the return of the cutter 180 to the original position is recognized by the sensor 185 disposed in the cutter 180 (S411), a buzz sound to tell the end of a sequence of treatment is issued from the speaker 110 (S412).

At this point, as shown in FIG. 29D, the adhesive is applied to the cut surface S and also the adhesive infiltrates into the joined portion C. The adhesive is also applied to the exterior surface of the body tissues L1, L2.

According to the present embodiment, as described above, the following effect is achieved.

A fluid such as blood generated from the body tissues L1, L2 in treatment can be introduced into the cutter guiding grooves 172, 174. Then, the fluid introduced into the cutter guiding grooves 172, 174 can be guided out of the energy treatment device 12b from the fluid discharge ports 186, 188 formed in the pipe 42 and the sheath 44 of the shaft 24. Thus, fluid can be prevented from remaining on joined surfaces of the joined portion C of the body tissues L1, L2 as much as possible and the body tissues L1, L2 can be treated for conjugation more quickly. Therefore, a sequence of treatment of joining the body tissues L1, L2 and coating the joined portion C can be provided more efficiently.

Not only the outer circumferential surface of the body tissues L_{T} to be joined is coated with the adhesive, but also the adhesive can be applied to the cut surface S of the body tissues L_{T} to coat contact surfaces with the adhesive so that fluid can be prevented from invading into the joined portion C of the body tissues L_{T}.

The hose 18a may be released to allow the adhesive to flow while the cutter 180 moves as described above, the hose 18a may be released after the movement regulation pin 42a of the pipe 42 reaches the other end 184b from the one end of 184a of the long groove 184 through the intermediate portion 184c. In this case, cutting of the body tissue L_{T} by the cutting edge 180a of the cutter 180 is completed (the cut surface S is already formed). Then, an adhesive is allowed to flow while the movement regulation pin 42a of the pipe 42 reaches the one end 184a from the other end 184b of the long groove 184 through the intermediate portion 184c. Then, a space is formed by the cut surfaces S of the body tissues L_{T} when the cutting edge 180a of the cutter 180 is drawn into the shaft 24 from the cutter guiding grooves 172, 174 of the first and second holding members 52, 54. If the adhesive is oozed out from the openings 192a, 194a, the adhesive enters the space between the cut surfaces S. Because the movement of the movement regulation pin 42a of the pipe 42 between the one end 184a and the other end 184b of the long groove 184 of the cutter 180 can be detected by the sensor 185, the spatial relationship between the body tissue L_{T} to be joined and the cutter 180 can easily be grasped. Thus, the timing to close the hose 18a can appropriately be set by the flow rate adjustment mechanism 134.

The present embodiment is described by assuming that a buzz sound is issued from the speaker 110, content of treatment or the procedure for treatment may be made known by voice. It is also preferable to make the first buzz sound and the second buzz sound vastly different so that which treatment is completed is easily recognizable.

The present embodiment is described by assuming a case of manually operating the cutter 180 by operating the cutter driving knob 34, but it is also preferable to cut the body tissues L_{T} by automatically operating the cutter 180 without operating the cutter driving knob 34 after treatment of the body tissues L1, L2 for conjugation by high-frequency energy is completed. That is, a sequence of treatment from the start of treatment using high-frequency energy to join the body tissues L1, L2 to the end of treatment to coat the joined body tissues L_{T} may automatically be performed.

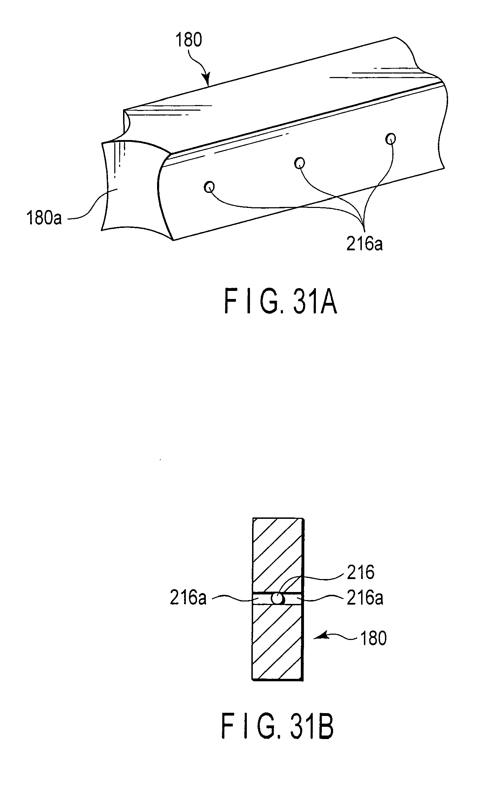

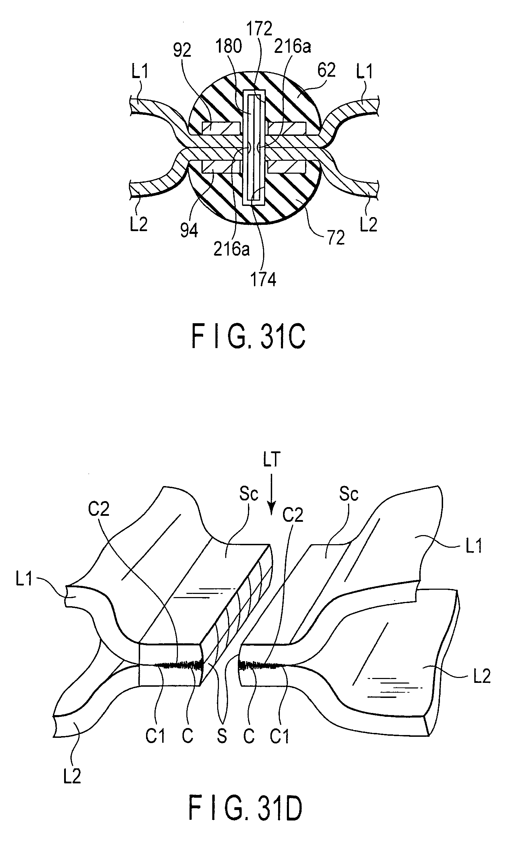

Next, a first modification of the third embodiment will be described using FIGS. 31A to 31D.

As shown in FIG. 31B, the cutter 180 has a duct 216 along the longer direction of the cutter 180 formed therein. The duct 216 formed inside the cutter 180 is connected to the hose 18a through the inside of the driving rod 182. The cutter 180 has a plurality of openings (conjugation maintenance assistance portions) 216a formed in the center in the width direction on the side surface. Thus, the adhesive is applied to the neighborhood of joint surfaces of the joined portion C simultaneously with cutting of the body tissue L_{T}. Therefore, the adhesive (conjugation adjunct) infiltrates to the joint surfaces of the joined portion C before being cured. In this case, as shown in FIG. 31D, an increasing amount of adhesive invades with a decreasing distance to the cut surface S and a decreasing amount of adhesive infiltrates with an increasing distance to the cut surface S.

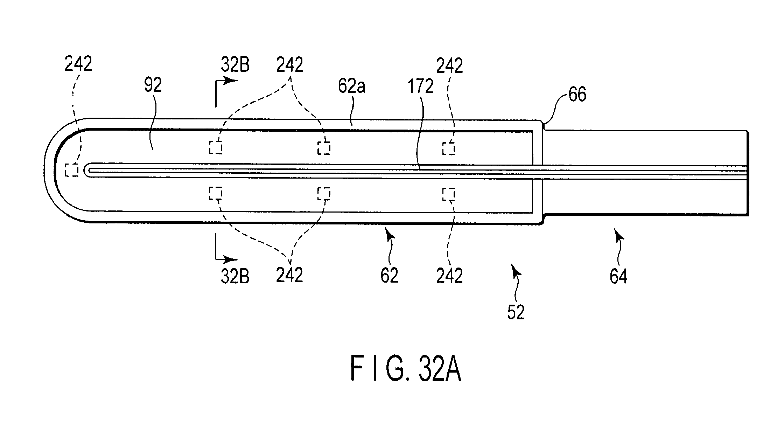

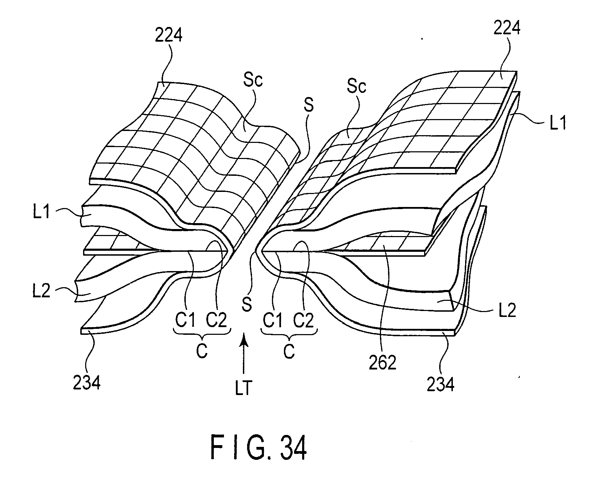

Next, a second modification of the third embodiment will be described using FIGS. 32A to 36.

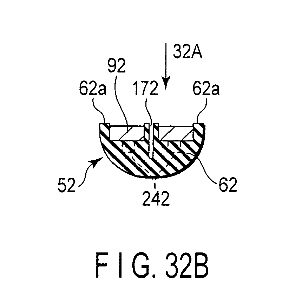

As shown in FIGS. 32A and 32B, first and second holding members 52, 54 include cutter guiding grooves 172, 174 formed therein. A cutter 180 (see FIGS. 28A and 28B) having a long groove 184 can be loaded into or unloaded from the cutter guiding grooves 172, 174.

A plurality of heaters (an energy output portion) 242 is disposed on a back surface of a high-frequency electrode 92 disposed on a main body 62 of the first holding member 52. Similarly, though not shown, a plurality of heaters (an energy output portion) 252 is disposed on the back surface of a high-frequency electrode 94 disposed on a main body 72 of the second holding member 54. The heaters 242, 252 can be controlled by a high-frequency energy output portion 104. That is, the high-frequency energy output portion 104 can supply energy not only to the high-frequency electrodes 92, 94, but also to the heaters 242, 252. Incidentally, the high-frequency energy output portion 104 may be made to be capable of selectively or simultaneously supplying energy to both the high-frequency electrodes 92, 94 and the heaters 242, 252.

The high-frequency electrodes 92, 94 are each formed from a material having a high thermal conductivity and thus, if the heaters 242, 252 are heated by supplying energy from the high-frequency energy output portion 104 to the heaters 242, 252, heat is conducted from the heaters 242, 252 to the high-frequency electrodes 92, 94. The heat conducted to the high-frequency electrodes 92, 94 is spread, for example, concentrically from the heaters 242, 252.

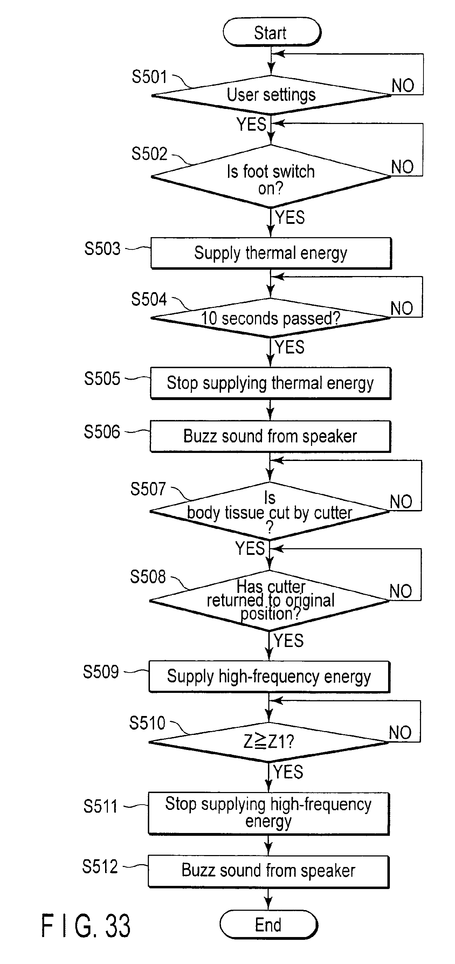

Next, the action of a medical treatment system 10 according to the present embodiment will be described using FIG. 33. Here, it is assumed that the nonporous sheet-shaped coating members 224, 234 containing a conjugation adjunct are used.

The amount of output from the high-frequency energy output portion 104 to the heaters 242, 252 and the output time are suitably set (S501). It is assumed here that the output time to the heaters 242, 252 is 10 sec.

If the pedal of the foot switch 16 is pressed (S502), energy is supplied from the high-frequency energy output portion 104 to the heaters 242, 252 so that the heaters 242, 252 are heated (S503). It is determined whether 10 sec has passed after the supply of energy is started (S504). After energy is output from the high-frequency energy output portion 104 to the heaters 242, 252 for 10 sec, the supply of energy to the heaters 242, 252 is stopped (S505). Then, a buzzer sound is emitted from a speaker 110 to tell the stop of the supply of thermal energy and also to tell that a cutter 180 will operate (S506).

A portion of the nonporous sheet-shaped coating members 224, 234 in contact with the high-frequency electrodes 92, 94 is melted after heat from the heaters 242, 252 are conducted to the high-frequency electrodes 92, 94.

Then, the cutter 180 is advanced to the cutter guiding grooves to cut body tissues L1, L2 (S507). That is, a cut surface S of the body tissues L1, L2 is formed. Then, the cutter 180 is returned to the original position thereof (S508).

Then, energy is supplied from the high-frequency energy output portion 104 to the high-frequency electrodes 92, 94 (S509). If an impedance Z is determined to have reached a threshold Z1 (S510), the output from the high-frequency energy output portion 104 to the high-frequency electrodes 92, 94 is stopped (S511).

A buzzer sound is emitted from the speaker 110 to tell the stop of the supply of energy (S512). Thus, a medical doctor or the like can make sure that a sequence of treatment has ended.

In the present embodiment, there is described a case when a sequence of treatment is carried out by combining both thermal energy by the heaters 242, 252 and high-frequency energy by the high-frequency electrodes 92, 94, but a sequence of treatment can be carried out using only thermal energy.

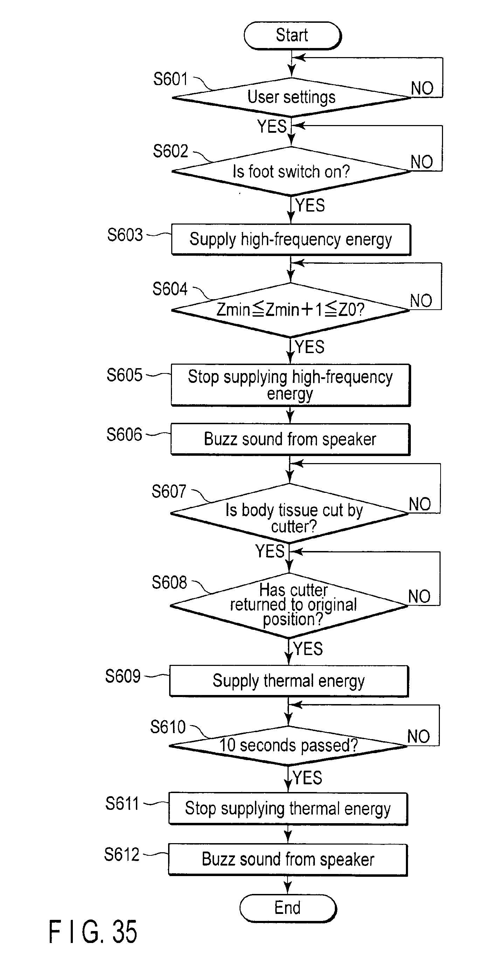

Next, the action (first action) of a medical treatment system 10 when the porous or mesh-shaped coating members 224, 234 containing a conjugation adjunct are used will be described using FIG. 35.

The amount of output of high-frequency energy or the like is set by operating a display unit 108 (5601). Then, the body tissues L1, L2 are held by the main bodies 62, 72 of the first and second holding members 52, 54 on which the coating members 224, 234 are disposed respectively. In this state, the pedal of the foot switch 16 is pressed (S602).

Energy is transmitted from the high-frequency energy output portion 104 to the high-frequency electrodes 92, 94 and a high-frequency current is passed through the body tissues L1, L2 (S603). When a high-frequency current is passed, the impedance Z drops from an initial value Z0 and then rises again (see FIG. 5A). When the impedance at the lowest point is Zmin and the next impedance measured after the impedance Zmin at the lowest point is Zmin+1, if the impedance Zmin+1 measured next is larger than the impedance Zmin at the lowest point and impedance Zmin+1 is smaller than the initial value Z0, the impedance Zmin at the lowest point can be determined (S604). If, as described above, the impedance Z is determined to rise again from the impedance Zmin at the lowest point, the supply of energy from the high-frequency energy output portion 104 is stopped (5605). At this point, a buzzer sound is emitted from the speaker 110 to tell the stop of the supply of high-frequency energy to the body tissues L1, L2 and also to tell that the cutter 108 will be operated (S606).

The cutter 180 slowly advances automatically along the cutter guiding grooves 172, 174 of the first and second holding members 52, 54 to cut the body tissues L1, L2 (S607) and returns to the original position thereof (S608). In this case, under control of a moving rate, position or the like, the cutter 180 moves from a state in which a movement regulation pin 42a of a shaft 24 is positioned in a locking portion 184a on the distal end side of a long groove 184 of the cutter 180 to a locking portion 184b on the proximal end side of the long groove 184, and moves again to be disposed in the locking portion 184a (original position) on the distal end side of the long groove 184.

Then, energy is supplied from the high-frequency energy output portion 104 to the heaters 242, 252 so that the heaters 242, 252 are heated (S609). When 10 sec passes after the supply of energy from the high-frequency energy output portion 104 to the heaters 242, 252 is started (S610), the supply of energy is stopped (S611).

The heaters 242, 252 conduct heat to the high-frequency electrodes 92, 94, and the high-frequency electrodes 92, 94 conduct the heat directly to body tissues, and thus, the body tissues (proteins) are integrally denatured and also fluid acting as a hindrance factor of linkage of proteins is removed.

Then, a buzzer sound is emitted from the speaker 110 to tell the end of a sequence of treatment (S612).

Incidentally, the coating members 224, 234 may be melted by generating heat into the body tissues L1, L2 using high-frequency energy or by directly applying heat using the heaters 242, 252.

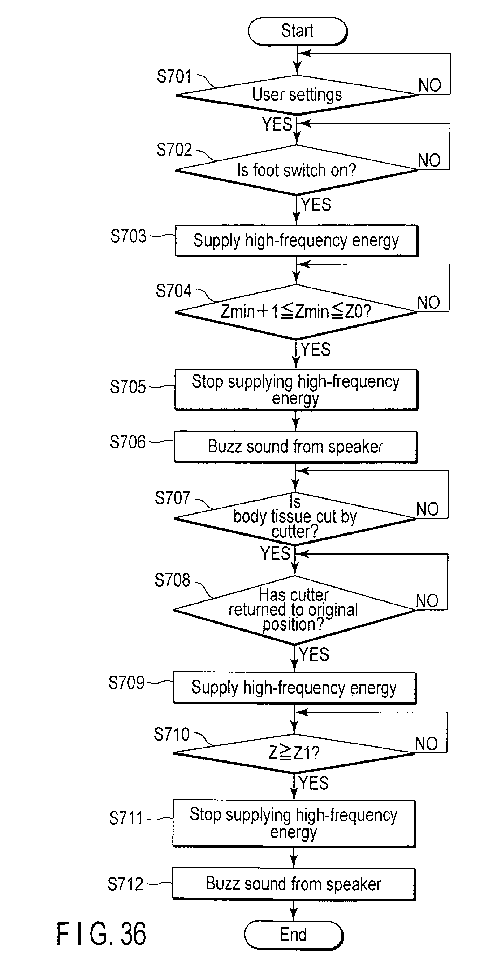

Next, the action (second action) of the medical treatment system 10 when the porous or mesh-shaped coating members 224, 234 are used will be described using FIG. 36.

Here, a case when, in contrast to the first action shown in FIG. 35, a sequence of treatment is carried out by high-frequency energy treatment by the high-frequency electrodes 92, 94 without using the heaters 242, 252.

Like the first action, the action is the same until the body tissues L1, L2 are cut by the cutter 180 to form a cut surface S (S701 to S708). After the cutter 180 is returned to the original position thereof, treatment using high-frequency energy is carried out by the high-frequency electrodes 92, 94 (S709). Then, if the threshold Z1 and the impedance Z are the same or the impedance Z is larger than the threshold Z1 (S710), the supply of energy from the high-frequency energy output portion 104 is stopped (S711). Then, the end of a sequence of treatment is told by emitting a buzzer sound from the speaker 110 (S712).

According to the present embodiment, as described above, the following effect is achieved.

Treatment of body tissues by high-frequency energy and treatment of body tissues by thermal energy can suitably be combined and thus, optimal treatment for the body tissues can be carried out.

### [Fourth embodiment]

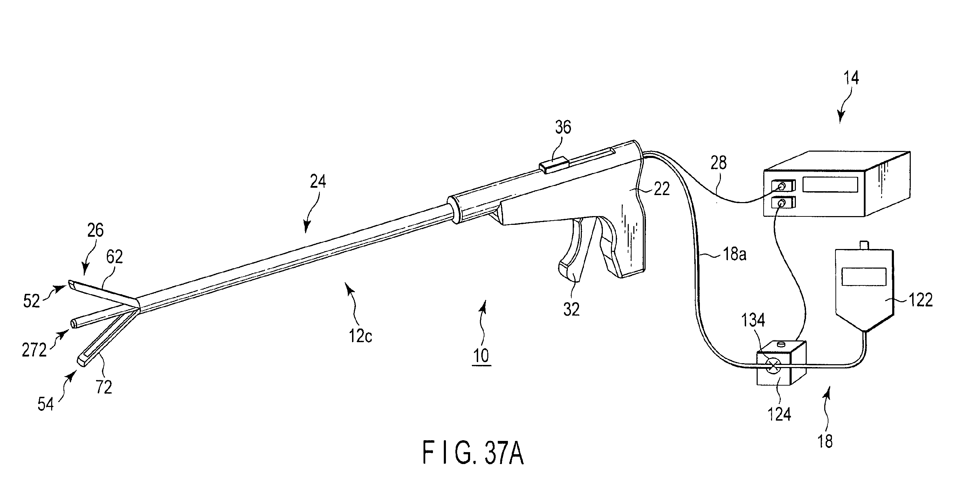

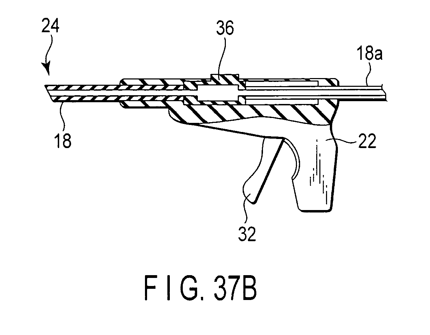

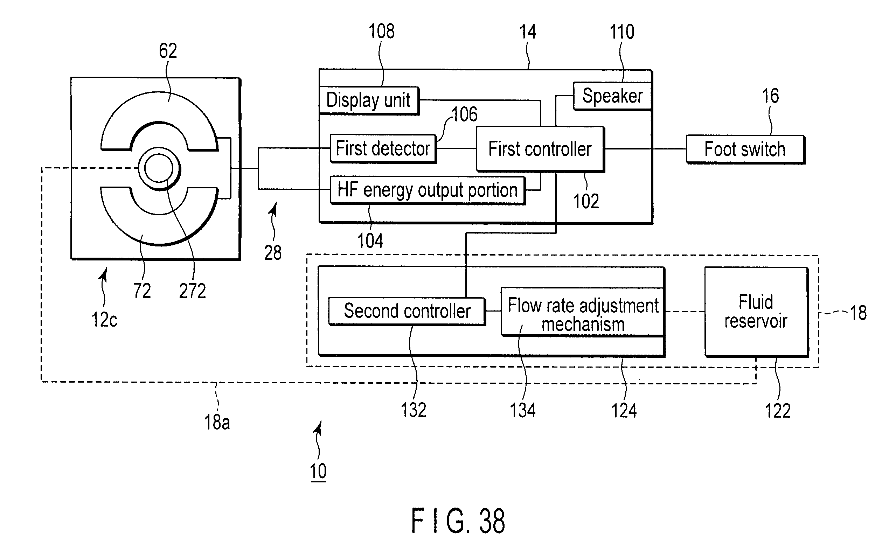

Next, the fourth embodiment will be described using FIGS. 37A to 41. The present embodiment is a modification of the first to third embodiments and the same reference numerals are attached to the same members as those described in the first to third embodiments or members achieving the same action as the action of those in the first to third embodiments and a detailed description thereof is omitted.

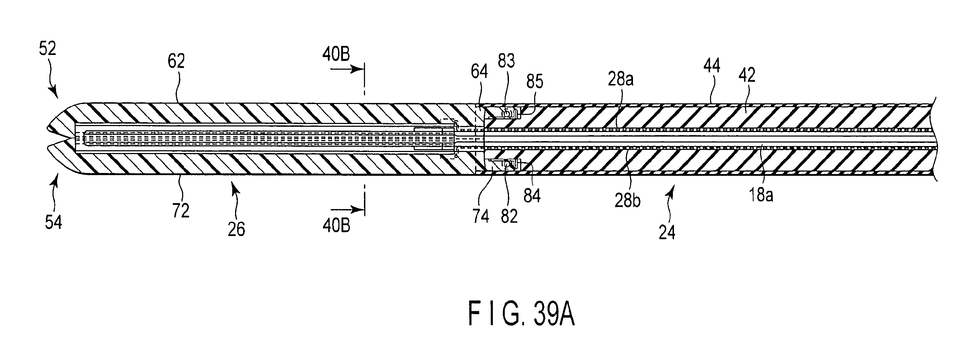

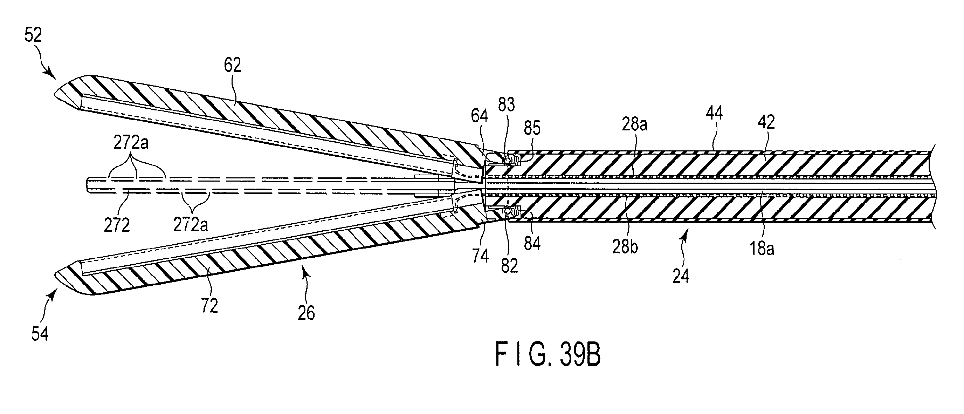

As shown in FIGS. 39A and 39B, a base 64 of a first holding member 52 is pivotally rotatably supported by a support pin 83 with respect to a pipe 42. The support pin 83 is disposed in parallel with a support pin 82 described in the first embodiment. The base 64 of the first holding member 52 is energized, like an elastic member 84 of a base 74 of a second holding member 54, by an elastic member 85 such as a plate spring. In the present embodiment, as shown in FIGS. 37A and 39B, both a first holding member 52 and a second holding member 54 of a treatment portion 26 of an energy treatment device 12c preferably open symmetrically with respect to the center axis of a shaft 24.

In the present embodiment, as shown in FIGS. 37A, 38, 39A, and 39B, a pipe-shaped member (join condition sustainment assistance portion) 272 is disposed as an auxiliary treatment device instead of a cutter 180 (see FIGS. 28A and 28B). The proximal end of the pipe-shaped member 272 is connected, as shown in FIGS. 39A and 39B, to a hose 18a.

As shown in FIG. 39B, a plurality of side holes 272a is formed on the side of a tip portion of the pipe-shaped member 272. The pipe-shaped member 272 can move between inside the shaft 24 and inside the treatment portion 26 by operating a pipe-shaped member movement knob 36 disposed on a handle 22 and can detect the position of the pipe-shaped member 272 relative to the treatment portion 26 or the shaft 24.

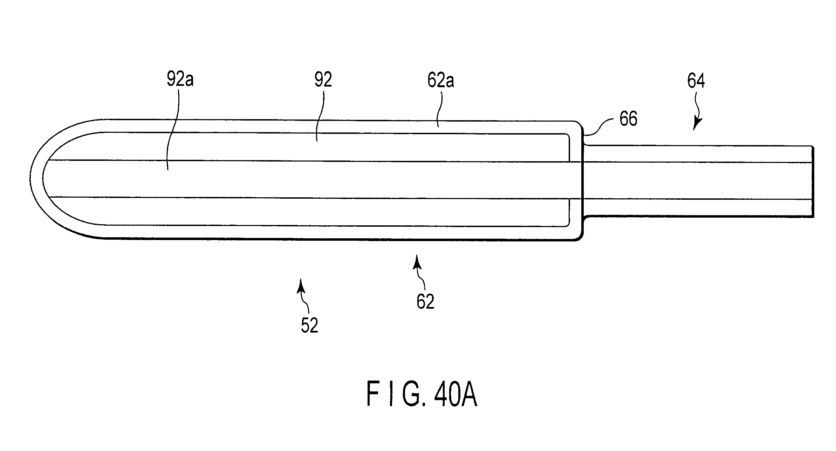

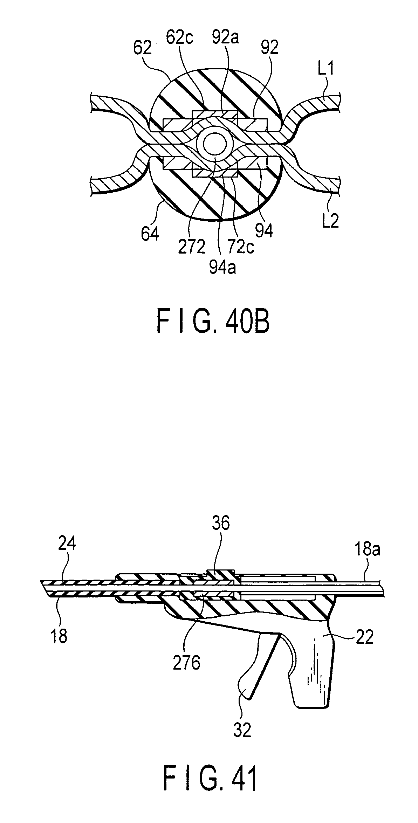

As shown in FIGS. 40A and 40B, a main body 62 of a first holding member 52 has a recess (pipe-shaped member guiding groove) 62c forming a space to move the pipe-shaped member 272 forward and backward formed therein. The width of the recess 62c is preferably formed slightly larger than an outside diameter of the pipe-shaped member 272. A high-frequency electrode 92a is also disposed on the recess 62c. The high-frequency electrode 92a disposed on the recess 62c and a high-frequency electrode 92c disposed on an inner side of a holding surface 62a of the main body 62 are at the same potential.

Incidentally, a recess 72c is also formed, as shown in FIG. 40B, in a main body 72 of a second holding member 54 and a high-frequency electrode 94a at the same potential as a high-frequency electrode 94 is disposed on the recess 72c.

Next, the action of a medical treatment system 10 according to the present embodiment will be described.

As shown in FIG. 40B, the pipe-shaped member 272 of the energy treatment device 12c is arranged between body tissues L1, L2 to be joined. Then, the body tissues L1, L2 are held by the main bodies 62, 72 of the first and second holding members 52, 54 and the pipe-shaped member 272 is sandwiched between the body tissues L1, L2. At this point, the mesh-shaped or porous coating members 224, 234 (see FIGS. 12B and 12C) containing a conjugation adjunct, described in the second modification of the first embodiment, are disposed outside the body tissues L1, L2 to be joined.

In this state, a substance (conjugation adjunct), such as an adhesive, that prevents fluid from infiltrating the body tissue L_{T} is introduced from a fluid reservoir 122 to the pipe-shaped member 272 through a hose 18a. Thus, the substance that prevents fluid from infiltrating the body tissue L_{T} is applied to the body tissues L1, L2 from the side holes 272a of the pipe-shaped member 272. In this state, the pipe-shaped member 272 is pulled out from between the main bodies 62, 72 of the first and second holding members 52, 54 by operating the pipe-shaped member movement knob 36. Thus, contact surfaces C1, C2 of the body tissues L1, L2 are in contact via the substance that prevents fluid from infiltrating the body tissue L_{T}.

Then, energy is supplied from a high-frequency energy output portion 104 to high-frequency electrodes 92, 94. Thus, the substance that prevents fluid from infiltrating the body tissue L_{T} on the joint surface is heated and also the joint surfaces are joined. The mesh-shaped or porous coating members 224, 234 melt due to heat from the body tissues L1, L2 to coat the outer side of the body tissues L1, L2 to be joined.

As more energy is supplied to the high-frequency electrodes 92, 94 or the supply of energy is stopped, the substance that prevents fluid from penetrating the body tissue L_{T} is hardened. At this point, the substance disposed on the joint surface of the body tissues L1, L2 to prevent fluid from penetrating the body tissue L_{T} penetrates from the contact surfaces C1, C2 of the body tissues L1, L2 toward the high-frequency electrodes 92, 92a, 94, 94a. Thus, the substance that prevents fluid from penetrating the body tissue L_{T} acts to sustain the joined state of the body tissues L1, L2.

According to the present embodiment, as described above, the following effect is achieved.

A fluid invasion prevention substance to the body tissue L_{T} can directly be applied to between the body tissues L1, L2. That is, the substance that reliably prevents fluid from penetrating the body tissue L_{T} can be applied to between the contact surfaces C1, C2 of the body tissues L1, L2. Thus, when the body tissues L1, L2 are joined using high-frequency energy or the like, since the substance that prevents fluid from penetrating the body tissue L_{T} is disposed between the contact surfaces C1, C2, even if a force to release joining of the body tissues L1, L2 acts, fluid can be prevented from penetrating the joint surface of the body tissues L1, L2 so that the joined state can be sustained.

Incidentally, a case when the coating members 224, 234 are used has been described in the present embodiment, but the coating members 224, 234 are not necessarily needed.

Also in the present embodiment, a case when the pipe-shaped member 272 is used, instead of the cutter 180, has been described, but an ultrasonic transducer 276 (see FIG. 41) may be disposed at the proximal end of the pipe-shaped member 272. That is, the pipe-shaped member 272 functions as an energy output portion that outputs ultrasonic energy to the body tissues L1, L2. In such a case, after pre-treatment to expose collagen to the contact surfaces C1, C2 of the body tissues L1, L2 by an ultrasonic device using the pipe-shaped member 272, the body tissues L1, L2 can be joined by the substance that prevents fluid from penetrating the body tissue L_{T}.

### [Fifth embodiment]

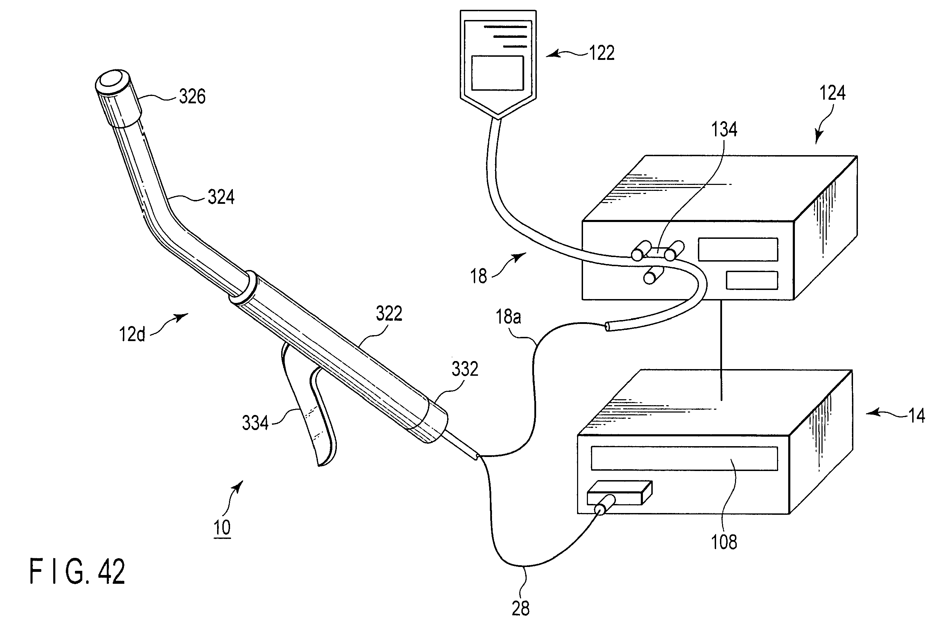

Next, the fifth embodiment will be described using FIGS. 42 to 45C. The present embodiment is a modification of the first to fourth embodiments. Here, a circular type bipolar energy treatment device (medical treatment device) 12d to carry out treatment, for example, through the abdominal wall or outside the abdominal wall is taken as an example of the energy treatment device.

As shown in FIG. 42, the energy treatment device 12d includes a handle 322, a shaft 324, and a treatment portion (holding portion) 326 which can be opened and closed. An energy source 14 is connected to the handle 322 via a cable 28 and also a fluid source 18 connected to the handle 322 via a hose 18a.

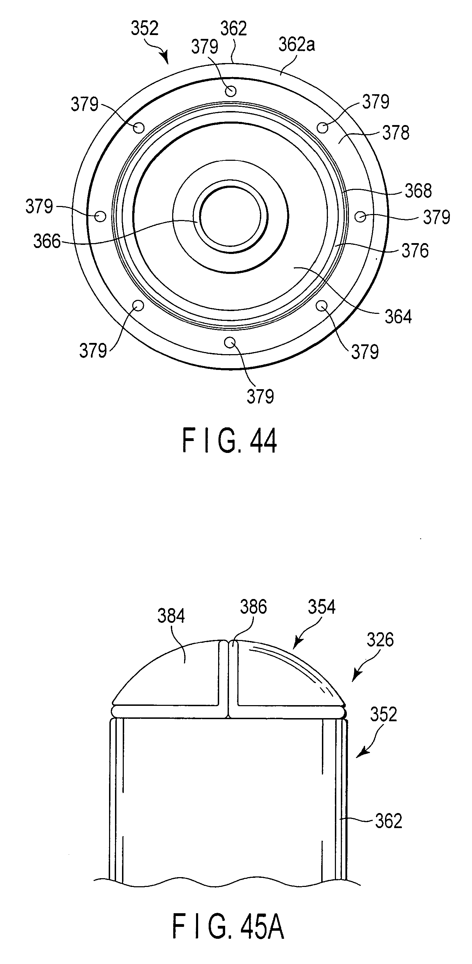

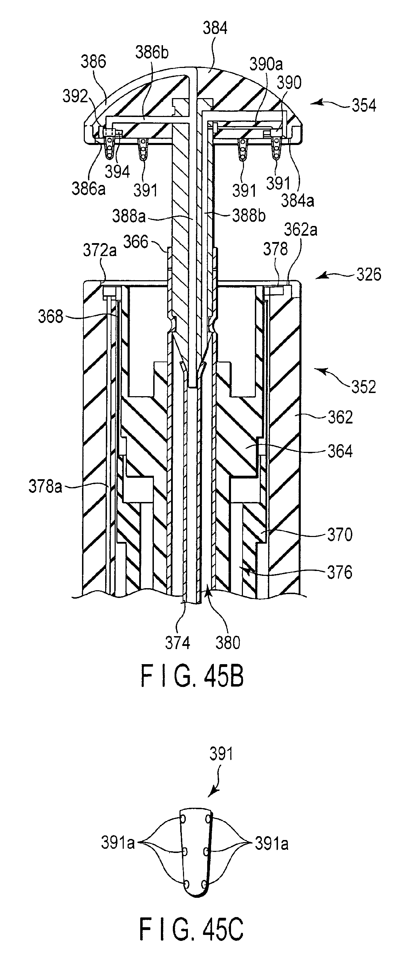

A treatment portion opening/closing knob 332 and a cutter driving lever 334 are disposed on the handle 322. The treatment portion opening/closing knob 332 is rotatable with respect to the handle 322. If the treatment portion opening/closing knob 332 is rotated, for example, clockwise with respect to the handle 322, a detachable-side holding member 354 described later of the treatment portion 326 is detached from a main body-side holding member 352 (see FIG. 45B) and if the treatment portion opening/closing knob 332 is rotated counterclockwise, the detachable-side holding member 354 is brought closer to the main body-side holding member 352 (see FIG. 45A).

The shaft 324 is formed in a cylindrical shape. In consideration of insertability into body tissues, the shaft 324 is made to be curved appropriately. It is, needless to say, that the shaft 324 is also suitably formed in a straight shape.

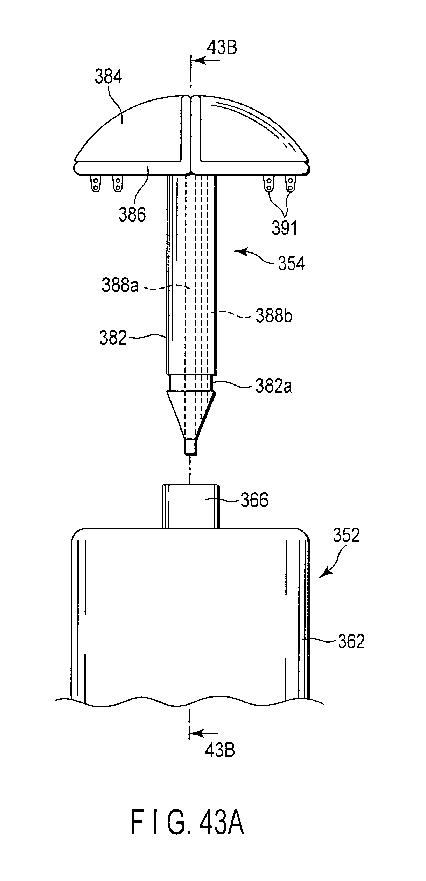

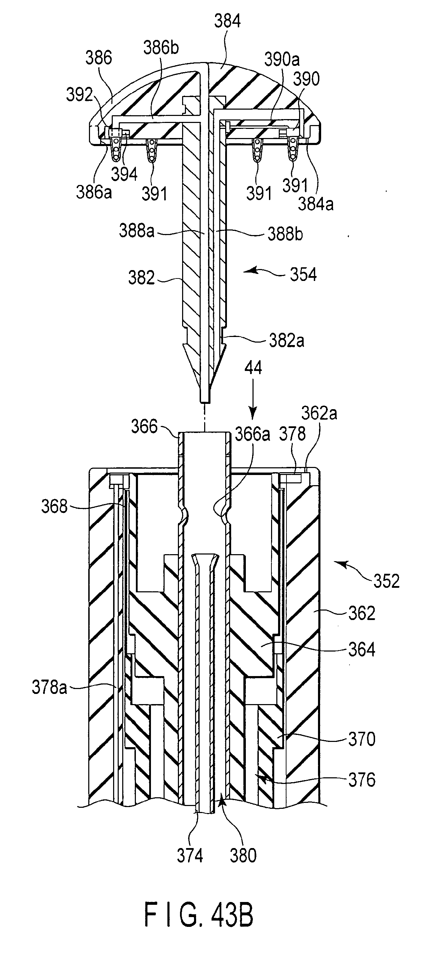

The treatment portion 326 is disposed at the distal end of the shaft 324. As shown in FIGS. 43A and 43B, the treatment portion 326 includes the main body-side holding member (first holding member) 352 formed at the distal end of the shaft 324 and the detachable-side holding member (second holding member) 354 detachable from the main body-side holding member 352.

The main body-side holding member 352 includes a cylinder body 362, a frame 364, an electrical connection pipe 366, a cutter 368, a cutter pusher 370, and a fluid duct 374. The cylinder body 362 and the frame 364 have insulating properties. The cylinder body 362 is coupled to the distal end of the shaft 324. The frame 364 is disposed in a state of being fixed with respect to the cylinder body 362.

The frame 364 has a center axis which is opened. The electrical connection pipe 366 is disposed in the opened center axis of the frame 364 movably within a predetermined range along the center axis of the frame 364. If the treatment portion opening/closing knob 332 of the handle 322 is rotated, as shown in FIGS. 45A and 45B, the electrical connection pipe 366 can move within the predetermined range through, for example, ball screw (not shown) action. The electrical connection pipe 366 has a projection 366a projecting inwards in a diameter direction formed thereon so that a connector 382a of an electrical connection shaft 382 described later can be engaged and released.

The fluid duct 374 to pass a fluid to the detachable-side holding member 354 is disposed inside the electrical connection pipe 366. Like the electrical connection pipe 366, the fluid duct 374 is movable within a predetermined range.

As shown in FIGS. 43B and 45B, a space is formed between the cylinder body 362 and the frame 364. The cutter 368 in a cylindrical shape is disposed in the space between the cylinder body 362 and the frame 364. The proximal end of the cutter 368 is connected to the tip portion of the cutter pusher 368a disposed inside the shaft 324. The cutter 368 is fixed to the outer circumferential surface of the cutter pusher 370. Though not shown, the proximal end of the cutter pusher 370 is connected to the cutter driving lever 334 of the handle 322. Thus, if the cutter driving lever 334 of the handle 322 is operated, the cutter 368 moves via the cutter pusher 370.

A first fluid airway (fluid channel) 376 is formed between the cutter pusher 370 and the frame 364. Also, a fluid discharge port (not shown) which is configured to discharge a fluid passing through the first fluid airway 376 to the outside is formed in the shaft 324 or the handle 322.

As shown in FIGS. 43B and 44, a first high-frequency electrode 378 in an annular shape is formed as an output member or an energy discharge unit at the tip end of the cylinder body 362. The tip end of a first electrical connection line 378a is fixed to the first high-frequency electrode 378. The first electrical connection line 378a is connected to the cable 28 via the main body-side holding member 352, the shaft 324, and the handle 322.

As shown in FIG. 44, recesses (conjugation maintenance assistance portion) 379 are formed in the first high-frequency electrode 378 at the tip of the treatment portion 326. Each of the recesses 379 is formed in such a way that a projection 391 (conjugation maintenance assistance portion, medical assistance portion) of a second high-frequency electrode 390 described later and disposed in the detachable-side holding member 354 is accepted in a non-contact manner.

An edge 362a of the cylinder body 362 is formed in a position higher than the first high-frequency electrode 378 on the outer side of the first high-frequency electrode 378. That is, the edge 362a of the main body-side holding member 352 is closer to a head portion 384 described later of the detachable-side holding member 354 than the first high-frequency electrode 378.

The length of the projection 391 of the second high-frequency electrode 390 of the detachable-side holding member 354 is formed to a height that does not come into contact with the recess 379 of the first high-frequency electrode 378 of the main body-side holding member 352. In other words, the depth of the recess 379 of the first high-frequency electrode 378 is formed deeper (longer) than the length of the projection 391 of the second high-frequency electrode 390. Then, the projection 391 forms a hole in the body tissues L1, L2, but does not necessarily need to cut through the body tissues L1, L2 and the tip (distal end with respect to the high-frequency electrode 390) of the projection 391 is suitably positioned closer to the high-frequency electrode 378 than the contact surfaces C1, C2 of the body tissues L1, L2.

The detachable-side holding member 354 includes the electrical connection shaft 382 having the connector 382a, the head portion 384, and a fluid duct 386. The head portion 384 is formed in a substantially semi-spherical shape. The connector 382a of the electrical connection shaft 382 is formed on the side closer to one end of the electrical connection shaft 382. The electrical connection shaft 382 has a circular transverse section, one end thereof is formed in a tapering shape, and the other end is fixed to the head portion 384. The connector 382a of the electrical connection shaft 382 is formed in a concave shape enabling engagement with the projection 366a of the electrical connection pipe 366 on the side closer to one end of the electrical connection shaft 382. The outer circumferential surface of a portion other than the connector 382a of the electrical connection shaft 382 is insulated by coating or the like.

The electrical connection shaft 382 has first and second ducts 388a, 388b formed so as to pass through one end and the other end thereof. The first duct 388a is formed to pass through the center axis of the electrical connection shaft 382. When the connector 382a of the electrical connection shaft 382 of the detachable-side holding member 354 is fitted to the projection 366a of the electrical connection pipe 366 of the main body-side holding member 352, the first duct 388a is communicatively connected to the fluid duct 374 of the main body-side holding member 352. The second duct 388b is communicatively connected to a second fluid airway (fluid channel) 380 between the electrical connection pipe 366 and the second fluid duct 374.

The head portion 384 has an edge 384a formed thereon. A second high-frequency electrode 390 in an annular shape is disposed as an output member or an energy discharge unit on the inner side of the edge 384a. One end of a second electrical connection line 390a is fixed to the second high-frequency electrode 390. The other end of the second electrical connection line 390a is electrically connected to the electrical connection shaft 382.

As shown in FIGS. 43B and 45B, the second high-frequency electrode 390 has a plurality of projections 391 disposed, for example, at equal intervals. If the detachable-side holding member 354 is brought closer to the main body-side holding member 352, the projection 391 can be disposed in a state in which the projection 391 is not in contact with the recess 379 of the first high-frequency electrode 378.

As shown in FIG. 45C, each of the projections 391 has one or a plurality of openings 391a formed therein. Each of the projections 391 preferably has a plurality of openings 391a formed therein. The projection 391 is communicatively connected to the first duct 388a and the second fluid duct 374 and can ooze out a fluid (conjugation adjunct) such as an adhesive through the opening 391a. The projections 391 are preferably disposed, for example, at equal intervals or in such a way that the same amount of liquid is oozed out from the opening 391a of each of the projections 391 by adjusting, for example, the diameter of the opening 391a.

A fluid discharge groove 392 in an annular shape is formed between the edge 384a of the head portion 384 and the second high-frequency electrode 390. The fluid discharge groove 392 is communicatively connected to the second duct 388b of the electrical connection shaft 382. The surface of the second high-frequency electrode 390 is in a state of being drawn to the edge 384a of the head portion 384. That is, the contact surface of the edge 384a of the detachable-side holding member 354 is closer to the main body-side holding member 352 than the second high-frequency electrode 390. Thus, vapor and liquids discharged from the body tissues L1, L2 in contact with the second high-frequency electrode 390 flow into the fluid discharge groove 392.

A cutter receiving portion 394 to receive the cutter 368 disposed on the main body-side holding member 352 is formed inside the second high-frequency electrode 390 in an annular shape.

Further, the fluid discharge groove 392 is communicatively connected to the head portion 384 and the second duct 388b of the electrical connection shaft 382. The second duct 388b is communicatively connected to the second fluid airway (fluid channel) 380 of the electrical connection pipe 366. The shaft 324 or the handle 322 has a fluid discharge port (not shown) that discharges the fluid having passed through the second fluid airway 380 to the outside formed therein.

Further, the fluid discharge groove 392 is communicatively connected to the head portion 384 and the second duct 388b of the electrical connection shaft 382. The second duct 388b is communicatively connected to the second fluid airway (fluid channel) 380 of the electrical connection pipe 366. The shaft 324 or the handle 322 has a fluid discharge port (not shown) formed to discharge a fluid flowing through the second fluid airway 380.

The electrical connection pipe 366 is connected to the cable 28 via the shaft 324 and the handle 322. Thus, when the connector 382a of the electrical connection shaft 382 of the detachable-side holding member 354 is engaged with the projection 366a of the electrical connection pipe 366, the second high-frequency electrode 390 and the electrical connection pipe 366 are electrically connected.

As shown in FIGS. 43A and 43B, the fluid duct 386 is disposed on the outer circumferential surface of the head portion 384 of the detachable-side holding member 354. The fluid duct 386 is disposed on the outer side of the edge 384a of the head portion 384. Then, as shown in FIGS. 43B and 45B, an opening (conjugation maintenance assistance portion) 386a is formed in a portion of the fluid duct 386 disposed on the outer side of the edge 384a of the head portion 384 and a branch duct 386b to discharge a fluid through the second high-frequency electrode 390 is formed inside the head portion 384. The fluid duct 386 is communicatively connected from the outer circumferential surface of the head portion 384 of the detachable-side holding member 354 to the first duct 388a inside of the electrical connection shaft 382. The branch duct 386b of the fluid duct 386 is communicatively connected to the first duct 388a to branch from the first duct 388a. The first duct 388a of the electrical connection shaft 382 is connected to the second fluid duct 374 disposed on the inner side of the electrical connection pipe 366 of the main body-side holding member 352.

The electrical connection pipe 366 is connected to the cable 28 via the shaft 324 and the handle 322. Thus, when the connector 382a of the electrical connection shaft 382 is engaged with the projection 366a of the electrical connection pipe 366, the second high-frequency electrode 390 and the electrical connection pipe 366 are electrically connected.

Next, the action of a medical treatment system 10 according to the present embodiment will be described.

As shown in FIG. 45A, the treatment portion 326 and the shaft 324 of the energy treatment device 12d are inserted into the abdominal cavity through, for example, the abdominal wall while the main body-side holding member 352 is closed with respect to the detachable-side holding member 354. The main body-side holding member 352 and the detachable-side holding member 354 of the energy treatment device 12d are opposed across body tissues to be treated.

The treatment portion opening/closing knob 332 of the handle 322 is operated to sandwich the body tissues L1, L2 to be treated between the main body-side holding member 352 and the detachable-side holding member 354. At this point, the treatment portion opening/closing knob 332 of the handle 322 is rotated, for example, clockwise with respect to the handle 322. Then, as shown in FIG. 45B, the electrical connection pipe 366 is moved to the side of the distal end portion thereof with respect to the frame 364 of the shaft 324 of the electrical connection pipe 366. Thus, the interval between the main body-side holding member 352 and the detachable-side holding member 354 increases so that the detachable-side holding member 354 can be separated from the main body-side holding member 352.

Then, the body tissues L1, L2 to be treated are arranged between the first high-frequency electrode 378 of the main body-side holding member 352 and the second high-frequency electrode 390 of the detachable-side holding member 354. The electrical connection shaft 382 of the detachable-side holding member 354 is inserted into the electrical connection pipe 366 of the main body-side holding member 352. In this state, the treatment portion opening/closing knob 332 of the handle 322 is rotated, for example, counterclockwise. Thus, the detachable-side holding member 354 is closed with respect to the main body-side holding member 352. In this manner, the body tissues L1, L2 to be treated are held between the main body-side holding member 352 and the detachable-side holding member 354.

In this state, the foot switch or hand switch is operated to supply energy from the energy source 14 to each of the first high-frequency electrode 378 and the second high-frequency electrode 390 via the cable 28. The first high-frequency electrode 378 passes a high-frequency current to the second high-frequency electrode 390 via the body tissues L1, L2. Thus, the body tissues L1, L2 between the first high-frequency electrode 378 and the second high-frequency electrode 390 are heated.

At this point, a fluid such as a vapor and a liquid arises from a heated portion of the body tissues L1, L2. The surface of the first high-frequency electrode 378 exposed to the side of the detachable-side holding member 354 is positioned slightly lower than the edge 362a of the main body-side holding member 352 while the first high-frequency electrode 378 is fixed to the main body-side holding member 352. Similarly, the surface of the second high-frequency electrode 390 exposed to the side of the main body-side holding member 352 is positioned slightly lower than the edge 384a of the head portion 384 of the detachable-side holding member 354 while the second high-frequency electrode 390 is fixed to the detachable-side holding member 354.

Thus, the edge 362a of the main body-side holding member 352 discharges a fluid arising from the body tissue L1 in contact with the first high-frequency electrode 378 to the second fluid airway 380 inside the electrical connection pipe 366 through the fluid discharge groove 392 and the second duct 388b. Also, the edge 384a of the detachable-side holding member 354 discharges a fluid arising from the body tissue L2 in contact with the second high-frequency electrode 390 to the first fluid airway 376 between the cylinder body 362 and the frame 364. Therefore, the edge 362a of the main body-side holding member 352 and the edge 384a of the detachable-side holding member 354 each serve the role as a barrier portion (dam) to prevent a fluid arising from the body tissues L1, L2 from leaking to the outside of the main body-side holding member 352 and the detachable-side holding member 354.

Then, while the main body-side holding member 352 and the detachable-side holding member 354 are closed, a fluid arising from the body tissue L1 flows into the first fluid airway 376 and a fluid arising from the body tissue L2 flows into the second fluid airway 380 by the edge 362a of the main body-side holding member 352 and the edge 384a of the detachable-side holding member 354 being kept in contact. Thus, a fluid arising from the body tissues L1, L2 is passed from the first and second fluid airways 376, 380 to the side of the handle 322 before being discharged to the outside of the energy treatment device 12d.

After the body tissues L1, L2 being joined, an adhesive is allowed to flow through a fluid reservoir 122, the hose 18a, the second fluid duct 374, the first duct 388a, and the branch duct 386b. Then, the adhesive is infiltrated to the joint surfaces of the joined portion C from the opening 391a of the projection 391 and cured. That is, an adhesive containing a conjugation adjunct is applied to the joint surfaces of the joined portion C of the treated body tissues L1, L2 and the joined portion C of the body tissue L_{T} is coated with the adhesive.

According to the present embodiment, as described above, the following effect is achieved.

Close contact of contact surfaces C1, C2 of the body tissues L1, L2 can be made more reliable by treating the body tissues L1, L2 for conjugation while an impedance Z of the body tissues L1, L2 is measured. After the body tissues L1, L2 are treated for conjugation, fluid can be prevented from invading into a joined portion C of a body tissue L_{T} treated for conjugation by coating the outer circumference of the body tissue L_{T} treated for conjugation with an adhesive or the like. Therefore, a state in which the contact surfaces C1, C2 of the body tissues L1, L2 are closely in contact (state in which the body tissue L_{T} is joined) can be sustained for a long time.

The present embodiment is described by assuming a case when the recess 379 of the first high-frequency electrode 378 and the projection 391 of the second high-frequency electrode 390, but similar treatment can be provided by disposing the mesh (see FIG. 6A) or porous (see FIG. 6B) conjugation assistance member 262 described in the first embodiment between the body tissues L1, L2.

The present embodiment is described by assuming a case of using the high-frequency electrodes 378, 390, but it is also preferable to use other types of energy such as heaters and laser light. In this case, the non-porous conjugation assistance member 262 (not shown) can be used. When the non-porous conjugation assistance member 262 is used, it is also preferable to the non-porous conjugation assistance member 262 by forming a hole using the projection 391.

Some embodiments have concretely been described with reference to drawings, but the present invention is not limited to the above embodiments and all embodiments that can be carried out without deviating from the scope thereof are included in the present invention.

### Industrial Applicability

According to the present invention, a medical treatment device capable of preventing fluid from invading to contact surfaces of body tissues and maintaining a state in which the joint surfaces are in close contact for a long time and a medical treatment system can be provided.

## Claims

1. A medical treatment device (12; 12a; 12b; 12c;
12d) configured to treat body tissues (L1, L2) for conjugation, **characterized by** comprising:
a pair of holding members (52, 54; 352, 354) to hold the body tissues;
an energy output portion (92, 94; 222, 232; 282, 284; 92, 94, 272; 378, 390) provided in at least one of the holding members to join the body tissues by supplying energy to the body tissues; and
a conjugation coating portion (162a; 262; 202; 204; 212a, 214a; 216a; 272a; 391a) to coat joint surfaces (C1, C2) of the body tissues with a substance capable of preventing fluid from invading.

2. The medical treatment device (12; 12a; 12b;
12c; 12d) according to claim 1, **characterized in that** the substance capable of preventing the fluid from invading contains a substance invading from the joint surfaces (C1, C2) of the body tissues (L1, L2) into the body tissues.

3. The medical treatment device (12; 12a; 12b;
12c; 12d) according to claim 1, **characterized in that** the substance capable of preventing the fluid from invading contains a gel or sheet bioabsorbable material (262) causing invasion to the joint surfaces (C1, C2) of the body tissues (L1, L2) to be joined.

4. The medical treatment device (12b, 12c; 12d) according to any one of claims 1 to 3, **characterized in that** the conjugation coating portion (212a, 214a; 216a; 272a) includes a medical assistance device (180; 202; 272; 391) that causes the body tissues (L1, L2) to be treated to discharge the substance capable of preventing the fluid from invading.

5. The medical treatment device (12b) according to claim 4, **characterized in that** the medical assistance device (180) includes a cutter (180) which is configured to cut the body tissues (L1, L2).

6. The medical treatment device (12; 12d) according to any one of claims 1 to 3, **characterized in that** the conjugation coating portion (204; 391a) includes at least a projection (202, 391) configured to puncture into the body tissues and
the projection includes at least an opening (204;
391a).

7. A medical treatment system (10), **characterized by** comprising:
the medical treatment device (12; 12a; 12b; 12c;
12d) according to any one of claims 1 to 6; and
an energy source (14) connected to the medical treatment device to supply energy to body tissues.

8. The medical treatment system (10) according to claim 7, **characterized by** further comprising: a fluid source (18) configured to store a substance capable of preventing fluid from invading into the body tissues (L1, L2) to be treated and configured to discharge the substance to the joint surfaces (C1, C2).

9. The medical treatment system (10) according to claim 7 or 8, **characterized in that** the energy source (14) can output and provide at least one of a high-frequency wave, a microwave, a heater, laser light, and ultrasonic energy to the body tissues (L1, L2) through the energy output portion in such a way that the body tissues are heated.

10. A medical treatment method to treat body tissues for conjugation, comprising:
holding the body tissues;
joining the body tissues by supplying energy to the body tissues; and
coating the body tissues with a substance capable of preventing fluid from invading from a neighborhood of joint surfaces to the joint surfaces.

11. A medical treatment method to treat body tissues for conjugation, comprising:
holding the body tissues;
denaturing the body tissues by supplying energy from a portion holding the body tissues;
cutting the body tissues after the body tissues being denatured;
joining the body tissues by supplying the energy from the portion holding the body tissues; and
coating the body tissues with a substance capable of preventing fluid from invading from a neighborhood of contact surfaces to the contact surfaces.

12. A medical treatment method to treat body tissues for conjugation, comprising:
holding the body tissues;
injecting an adhesive into the held body tissues;
and
dehydrating and drying the body tissues.

13. A medical treatment method to treat body tissues for conjugation, comprising:
holding the body tissues;
injecting an adhesive into the held body tissues;
and
dehydrate the held body tissues to dry the body tissues.
